# EUROPEAN PATENT SPECIFICATION

(11) **EP 4 558 808 B1**
(45) Date of publication and mention of the grant of the patent: **31.12.2025**
(21) Application number: 23758586.4
(22) Date of filing: 17.08.2023
(51) Int. Cl.: G01N 21/03, G01N 21/11, G01N 15/00, G01N 11/04, G01N 15/14, G01N 33/00, G01N 1/00, G01N 15/1434, G01N 15/0227

(54) **INSERTION, MEASURING CUVETTE, DEVICE AND METHOD FOR POWDER CHARACTERIZATION**
EINSATZ, MESSKÜVETTE, VORRICHTUNG UND VERFAHREN ZUR PULVERCHARAKTERISIERUNG
INSERTION, CUVETTE DE MESURE, DISPOSITIF ET PROCÉDÉ DE CARACTÉRISATION DE POUDRE

(30) Priority: 18.08.2022 EP 22382790
(43) Date of publication of application: 28.05.2025
(73) Proprietor: Blanco Fernández, David, 50410 Cuarte de Huerva, Zaragoza (ES)
(72) Inventor: Blanco Fernández, David, 50410 Cuarte de Huerva, Zaragoza (ES)
(74) Representative: Isern Patentes y Marcas S.L.
(86) International application number: PCT/EP2023/072690
(87) International publication number: WO 2024/038147

(56) References cited:
- US-B2- 8 121 388
- BLANCO DAVID ET AL: "Image-based characterization of powder flow to predict the success of pharmaceutical minitablet manufacturing", INTERNATIONAL JOURNAL OF PHARMACEUTICS, ELSEVIER, NL, vol. 581, 27 March 2020 (2020-03-27), XP086149826, ISSN: 0378-5173, [retrieved on 20200327], DOI: 10.1016/J.IJPHARM.2020.119280

## Description

### FIELD OF THE INVENTION

The invention refers to the field of material science and engineering, more specifically, to powder technology. The invention is applied to monitor and control the behaviour at interfaces resulting from surface modification by dry powder coating.

### PRIOR ART

Surface modification and functionalisation of powdered materials are used to maximise product and process performance. Dry powder coating is a well-stablished process in pharmaceutical development to alter the surface properties and/or functionality of particles by placing smaller guest particles (0,1-50 µm) on larger host/carrier particle surfaces (1-500 µm). For instance, oral solid dosage forms (OSD) manufacturing requires a series of unit operations, including blending, roller compaction, granulation, capsule filling and tablet manufacturing. In these processes, frictional forces between particles (internal friction) and between powder and processing equipment (wall friction) often generate mechanical stress on the instruments and may compromise the quality of the final product. Therefore, the addition of lubricant agents to the formulation is necessary to obtain high quality products with an efficient and trouble-free processing.

Despite the large variety of lubricants, magnesium stearate (MgSt) is by far the most used as cost-effective, non-toxic compound with excellent anti-adherent properties. Classified as a boundary layer lubricant, MgSt is mechanochemically absorbed into surfaces thereby reducing the shear ratio between the powder-tool interfaces and particle-particle interfaces thus accommodating motion and shear. During blending, free lubricant agglomerates are at first adsorbed on the surfaces of the excipient and drug particles, and thereupon, the shear forces induce mechanical deagglomeration and delamination over the carrier particles. Thus, dry powder coating with MgSt leads to a variety of physicochemical changes in the formulation and subsequent tablet properties.

Material attributes and especially processing conditions have a major influence on the performance of dry powder coating processes. For instance, an excess of MgSt and/or overblending, i.e., overlubrication, interferes with the bonding properties of the carriers by acting as a physical barrier decreasing the effective bonding surface area during compaction. This is attributed to the formation of weaker bonds between lubricant molecules rather than strong bonds between excipient molecules, which reduces the compactability and mechanical strength or hardness of tablets. Therefore, tensile strength (TS) is widely used as a standard test for overlubrication or descriptor of the extent of the lubricant coating after compaction, whereby the mechanical properties of the formulation excipients determine how much it is affected. Plastically deforming materials are generally more sensitive to the MgSt coating than fragmentary, as these cannot create new non-lubricated contact surfaces under pressure. As a rule, considering that MgSt gives acceptable lubricity when poorly distributed and to minimize the expensive consequences of overlubrication, short blending times (2-5 minutes) and low amounts (0.5-1% w/w) are typically used in pharmaceutical development. Furthermore, according to the previous operating standards, several authors recognize that irregular powder morphologies, as opposed to granules, prevent the MgSt from blending uniformly and the coating is often discontinuous.

Similarly, the MgSt coating modifies particle surface properties, such as size, shape and roughness. Thus, powder flowability is affected as MgSt modifies the area of contact between the sliding interfaces, the type and strength of the attractive forces between the contacting surfaces, and the shearing and rupture of the materials at the contact points and the surrounding area during sliding. The effect of MgSt addition on formulation properties has been widely evaluated using state-of-the-art flow measuring techniques such as angle of repose, avalanche time, shear cell and powder rheometry, i.e., both static and dynamic measurements and different shear conditions. The consensus is that MgSt improves flowability (glidant effect) to a plateau, which only decreases in some materials as the lubricant concentration and/or blending time increase significantly, i.e., far from standard operating ranges. This flowability improvement is recognized to be affected principally by the particle surface roughness. Furthermore, it depends on the formulation properties and the measuring technique and is more pronounce at larger particle sizes (see reference Morin, G., & Briens, L. "The effect of lubricants on powder flowability for pharmaceutical application". AAPS PharmSciTech, 2013, 14(3), 1158-1168.).

Overlubrication and related problems are well-known in the manufacture of OSD forms. There have been numerous attempts to detect lubricant films by chemical or physical methods in order to get better understanding of the nature of these films from intermediate products. Traditional methods such as high-performance liquid chromatography, energy-dispersive X-ray microanalysis, and secondary-ion mass, inductive coupled plasma, ultraviolet or Raman spectroscopies have been adopted to monitor lubricant blend uniformity, but these analyses demonstrate low reliability, are time-consuming and demand expertise and precision. Conversely, near-infrared (NIR) and laser-induced breakdown (LIBS) spectroscopies have succeeded in the quantitative determination of MgSt coating from powders and tablets. NIR measurements prove to be a well-suited technique to detect and quantify MgSt among other quality attributes of intermediate products. Thus, these techniques can determine the extent of the lubricant coating but do not describe the modified behaviour at interfaces. To date, end-product testing and/or tablet QA remain the first choice to determine the endpoint of the lubricant blending process in pharmaceutical development, with limited understanding of the process and considerable time and resources consumption for risk assessment of overlubrication.

Document FI 128765 B discloses an arrangement of a device for a general powder flow classification using cellulose-based commercial excipients (MCC). Said device including a cuvette which comprises two cavities connected by an open aperture and characterized in that the bottom of the cuvette may be either transparent or opaque, depending on the imaging method. Furthermore, an applied force to said cuvette causes the powder mass to impact with a pin and split in two parts. Then, binary image processing determines the symmetry of the powder mass after the impact to characterize the flowability.

Similarly, Blanco, D., et al. "Image-based characterization of powder flow to predict the success of pharmaceutical minitablet manufacturing", Int. J. Pharm. 2020. 581(May), 119280; shows an embodiment of the device to simulate and/or reproduce the die-filling process using a wide variety of pharmaceutical excipients. Also, Blanco, D., et al. "Effect of colloidal silicon dioxide and moisture on powder flow properties: Predicting in-process performance using image-based analysis". Int. J. Pharm. 2021. 597(March), 120344; further reported the use of the previous mentioned embodiment of the device to simulate the die-filling process. Said device characterizes the strong effect on powder flow properties of cohesive lactose resulting from the uniform nanoparticle deposition of a flow additive at different amounts (constant blending time of 10 minutes).

It follows from the above that the prior art does not provide a detailed description of the extent of the MgSt coating in powders by examining the behaviour at interfaces under low-shear flow conditions.

### DESCRIPTION OF THE INVENTION

Particle size, density, surface area, smoothness of the surface and particle-to-particle interactions are amongst the most important powder properties that affect process performance. Dry powder coating with MgSt affects all these parameters and can lead to adverse effects on the final product. Spectroscopic techniques minimise or prevent these by characterising the particle surface properties, but direct characterisation of the modified behaviour at interfaces through flowability measurements remains in many respects an analytical challenge. State-of-the-art flow techniques recognize that the effect on flowability from MgSt addition largely depends on particle surface roughness, which is gradually smoothen, thus increasing flowability. For instance, dynamic flow measurements have been reported to be more sensitive to the MgSt coating and provide more accurate and reproducible results than static measurements. These flowability differences between testing conditions therefore suggest that the impact of the lubricant in the interaction forces cannot be only attributed to the physical contact between coated surfaces that dominate static flow measurements, but also to the cohesive vdW forces that have more influence in dynamic flow regimes.

Considering that MgSt increases the contact surface between particles and based on the irregular stratified morphology of the aggregates, the flowability of a powder should be worsen rather than improved regardless the glidant effect that the addition of small MgSt particles may have. Therefore, it is assumed that the prevalence of gravitational forces during the analysis of bulk powder volumes promotes flowability and prevents a detailed description of the most likely particle-level cohesion phenomena associated to the MgSt coating. This is confirmed from the results obtained with the novel device of the invention which, in contrast to an increase in flowability, shows a strengthening of the interaction forces at interfaces (e.g., friction and vdW forces) that restrict powder flow. Thus, the sustained increase in flowability from conventional flow analysis can be explained by the known ability of MgSt particles to delaminate under an applied force, e.g., gravity. Moreover, these differences in flowability show that the device of the invention describes the modified behaviour at particle-particle interfaces due to the MgSt coating instead of bulk powder flow properties.

**In** fact, the arrangement of a device disclosed in the prior art overcomes bulk-derived gravitational effects, but the analysis is limited to flow characterization and does not consider such slight changes in particle surface characteristics. Thus, said device is effective to measure the improvement in flowability by the addition of colloidal silicon dioxide (CSD), as this nanoparticulate material produces significant changes in the surface roughness of poor flowing lactose. That is, after blending for 10 minutes (standard CSD blending time), the spherical nanoparticles are uniformly distributed within the powder mass and the effect on flowability depends on the amount of CSD added, the effect on particle roughness and the free available fraction if the amount is too high. In contrast, the coating of MgSt is typically discontinuous and it forms gradually over blending time. Furthermore, according to pharmaceutical development recommendations, the end point for the blending unit operation corresponds to short blending times and low amounts of lubricant to prevent overlubrication phenomena. Then, the progressive delamination and/or coating by MgSt is very limited, resulting in very subtle changes in the surface characteristics of substrate particles and related behaviour at interfaces. Therefore, prior art flow techniques cannot be considered for risk assessment of the MgSt blending unit operation.

To overcome the afore-mentioned problem, this patent application provides an insertion, measuring cuvette, device and method to monitor and control the extent of the MgSt coating in intermediate powders, even if small MgSt amounts are added or short blending times are considered. Their combination characterizes the non-uniform distribution of MgSt within the powder mass as the merged interaction of the lubricated powder sample (both lubricant and powder particles), thus identifying the endpoint for the MgSt blending. In particular, the invention relates to the measurement of the shear strength or resistance to flow in a powder sample according to the governing interaction forces in said sample, i.e., according to dynamic interactions and static contacts measured at different (low) shear strengths. This customized shear strength measurement depends on the physical properties of the material, the geometry of the insertion, the configuration of the movement in the device and the applied method. Said combination is complemented by the extensive reduction of bulk-derived gravitational effects by examining small amounts of powders. Thus, the invention monitors and controls MgSt overlubrication phenomena at reduced cost for safer manufacturing of pharmaceuticals.

In addition to the foregoing attributes, the invention possesses numerous benefits and advantages over known flowability methods. In particular, the invention facilitates the dispersion of a small amount of powder sample. In addition to the natural tendency of particles to separate by size during movement, the dispersion depends on the magnitude of the interaction forces between particles which can also be minimized by limiting the shear strength in the powder sample. For instance, for physical characterisation of powders using image analysis technologies, adequately dispersed samples are needed to provide consistent and repeatable analysis. Advantageously, the novel device of the invention includes topographic and/or photometric stereoscopic imaging to powder flow analysis. Thus, the invention further extends the characterization of powders by connecting certain physical properties of the powder sample, such as particle size distribution (PSD) and morphology, to its processability. Moreover, the invention can be applied to non-cohesive and cohesive powders, provides fast and effective measurements in a laboratory or industrial process environment setting and is recognized as a rapid non-destructive and sample-sparing analysis.

Some industrial applications of the invention comprise powder processing industries, including pharmaceuticals, fine chemicals, food, cosmetics, toners, metals, ceramics, plastics, powder coatings, cements and additive manufacturing (AM). For instance, for particle separation and processing, understanding and controlling the surface properties and behaviour at interfaces is a crucial task. In pharmaceutical technology this applies to process operations such as blending, coating and conveying of powders, and to applications such as dry powder inhalers.

A first aspect of the invention refers to a detachable insertion according to claim 1 for cooperating with a measuring cuvette, the measuring cuvette being suitable for receiving a powder sample.

The geometry of the insertion plays a crucial role in characterising the different interaction mechanisms according to the physical properties of the powder sample. Thus, the powder flow pattern in the device of the invention is determined primarily by the acceptance angle of the funnel portion and the shape and size of the outlet. Said "powder flow pattern" refers to the resulting geometry of the dispersed powder sample in the device generated after flowing through the outlet of the insertion. Advantageously, the inlet of the insertion allows a tongue-and-groove connection with a feeding accessory, as later explained. Throughout this document, the concept of having two elements connected in "flow communication" means that transfer of the powder sample is possible between said elements.

An "inert material" refers to a material that complies with the cleaning procedures used in the powder processing industries. Preferably, the inert material has a smooth surface that does not physically and/or chemically interact with the powder sample, preventing powder sticking and material incompatibilities, respectively. Furthermore, preferably, the material with a smooth surface refers to a low- coefficient of friction material (COF), in the range COF < 0.55, which minimizes and standardizes undesired interactions between the powder sample and the insertion to ensure repeatability. Additionally, the inert material has a low moisture absorption to support the cleaning process between measurements (typically using water and pressured air). Examples of inert materials are stainless steel or a steel alloys, aluminium or an aluminium alloys, titanium, polyetheretherketone (PEEK), fluoropolymer (PTFE, ETFE, PVDF, etc), polyethylene (PE), polypropylene (PP), polyamides (PA, nylon), Bakelite-based polymers, polystyrene (PS), polycarbonate (PC), biodegradable polymers (PLA, PHBV, PCL) and/or composites or coating materials thereof.

Advantageously, the insert can be made of pharmaceutical grade stainless steel (AISI 304, 304L, 326, 316L or alloys), which has more consistent surface roughness (e.g., measured as the arithmetic average of surface heights, often abbreviated Ra, in the range Ra ≤ 0.8 µm) and COF (0.27-0.52) than plastic polymers, thus maximizing the above-mentioned attributes of an inert material. Moreover, 316L grade stainless steel is preferred for manufacturing equipment in the powder processing industry, so an insertion made of it better simulates the wall friction.

In a preferred embodiment, the insertion is characterized in that:
- the funnel portion defines an acceptance angle between 15-50°; and/or
- the shape of the dome-shaped portion substantially exhibits a semi-circular or comprises at least one curved segment; and/or
- the diameter of the dome-shaped portion is within the range of 8-18 mm or, in case it comprises a curve or in case of containing a curved segment, the chord of said curve or segment is within the range of 8-18 mm; and/or
- the length of the funnel portion lies within the interval 10-35 mm; and/or
- the outlet substantially exhibits a quadrilateral, circular shape or a combination thereof.

Preferably, in this preferred embodiment, the outlet of the insertion has a quadrilateral shape, the fourth side being understood as that defined by a base of a measuring cuvette. That is, that the outlet comprises an orifice and not an open aperture. Advantageously, this geometry increases the precision between measurements, as it adapts to the geometry of the narrower part of the funnel portion thus avoiding flow interference in the powder sample during analysis. Moreover, this geometry facilitates machining, thus reducing production costs. The size for the quadrilateral shape is less than 8 mm on side or, in case of containing any curve or semicircle the chord and/or diameter is less than 8 mm.

More advantageously, the dome-shaped portion of the insertion facilitates a natural and reproducible accommodation of the powder sample when the novel device of the invention is accelerated during the measurement, thereby increasing reproducibility and repeatability. Moreover, this geometry further allows the implementation of a consolidation step which is discussed below. In addition, the acceptance angle (α) defined by the funnel-shaped portion corresponds to the diameter of the dome-shaped portion or the chord in case of a curved shape on one side and determines the size of the insertion outlet on the other. Thus, α determines the flow regime or magnitude of shear strength between particles.

A second aspect of the present invention relates to a measuring cuvette according to claim 3 for cooperating with the detachable insertion, the measuring cuvette being suitable for analysing a powder sample.

Throughout this document, "translucent material" is defined as an inert material whose molecular density is variable and modulates the amount of light that can pass through. Advantageously, this material acts as a diffuser of the applied lighting achieving a continuous and uniform illuminated surface and/or certain homogeneity in the distribution and intensity of the lighting. More advantageously, the translucent material can be non-sticky, i.e., reduce the adherence of powder particles by a low surface tension, and has a low coefficient of friction (COF < 0.35), which is preferred for the base of the measuring cuvette of the invention to maximize the flow of the powder sample. Therefore, transparent materials that prevent homogeneous illumination of the powder sample and require additional image post-processing steps to obtain quality images are avoided. Examples of translucent materials include the above-mentioned inert materials meeting the requirements of a translucent material such as PE's and fluoropolymer's, and others such as polyacrylates (PAN, PMMA, etc), ceramic and glass materials.

Preferably, the insertion and the measurement cuvette are made of non-translucent inert materials through which light cannot transmit. **In** this way, the illuminated base containing the powder sample can be clearly distinguished from the surrounding area during the analysis with the device. Advantageously, the base can be easily removed from the measuring cuvette, which facilitates cleaning of the cuvette between measurements and reduces testing time. Moreover, the measuring cuvette is partially and/or totally protected from the external (ambient) illumination so that ambient light exposure is minimized or avoided, thus only the lightning system(s) in the device illuminates the powder sample in the measuring cuvette.

The fixed portion of the loading chamber exhibits geometrical dimensions such that it substantially fits with the insertion of the invention, i.e., the fixed portion of the measuring cuvette and the insertion are complementary and can be coupled with each other. **In** this way, the insertion of the loading chamber is interchangeable. Advantageously, this simplifies the assembly and its associated costs by reusing the measuring cuvette while varying the unlimited array of geometries of the insertion. This also allows the replacement of possible damage to the individual components and further investigation and/or acquisition of prospective geometrical arrangements of the insertion. In this way, the insertion in the loading chamber can be replaced according to analytical requirements. More advantageously, separating the insertion from the measuring cuvette facilitates the cleaning of the assembly. This avoids cross contamination and, in the case of lubricated powder samples, facilitates the difficult task of removing the small particles mechanochemically adhered to the surfaces of the insertion during measurements. Moreover, the insertion and the measuring cuvette can be composed of materials with different properties, which combined maximize the processing performance of powders using the device.

In a preferred embodiment of the measuring cuvette:
- the measuring cuvette and the base are made of antistatic materials; and/or
- at least one of the disturbance pins is cylindrical with a base diameter between 2 and 8 mm, and said at least one of the disturbance pins is made of an inert material and is adapted to be aligned with the outlet of the insertion and the funnel portion.

Throughout this document, an "antistatic material" is understood as an inert material having a surface resistivity greater than 1x10⁶ ohms/square, thus both antistatic and insulative properties are considered. These antistatic materials reduce, dampen or inhibit the static electricity within the measurement system, which is built by particle-particle or particle-equipment interactions (i.e., triboelectric charging) or by transfer from the environment, for example, when handled by the operator. Therefore, the inclusion of these materials minimizes the possible adverse effects of electrostatic repulsion between powder particles that often occurs when handling small volumes of powder and may compromise the analysis. Examples of antistatic materials include some of the above-mentioned inert materials and other plastics such as acetal (POM), ABS, methacrylate, PFTE, polyether ketone (PEK), and polymers containing carbon, PFTE, nylon or graphite powders to made them antistatic.

The antistatic nature of the base and the measuring cuvette electrically insulate the insertion, so said insertion can be made of conductive material stainless steel for the advantages mentioned above. Thus, the fixed portion of the loading chamber containing the insertion and preferably the entire measuring cuvette are made of antistatic materials. Optionally, the fixed portion of the loading chamber and the base can be made of antistatic material, which allows the insert and other parts of the measuring cuvette to be made of stainless steel. Furthermore, the pin(s) can be made of an inert material and must be able to disturb the powder sample after flowing through the outlet. It can be integrated into the base or cannot be part of said base and be part of the measuring cuvette. Advantageously, when integrated into the base, the pin(s) can also be made of stainless steel as it is electrically insulated. Hence, all surfaces that may become charged after cleaning with water and air between measurements may be antistatic or, if conductive (surface resistivity less than 1x10⁶ ohms/square), may be electrically insulated to avoid charging after cleaning. Another preferred embodiment involves the manufacture of the insertion or alternatively the fixed portion of the measuring cuvette, and the base using antistatic materials, which allows a measuring cuvette partially made of stainless steel.

In other preferred embodiment of the measuring cuvette, the base comprises a plate of non-sticky translucent material, preferably a fluoropolymer such as polytetrafluoroethylene.

Advantageously, the base can be made of PFTE, which combines excellent translucid and antistatic properties of an inert material and is approved by the Food and Drug Administration (FDA). More advantageously, PFTE has a very low static and kinetic COFs (0.04) which as previously mentioned is advantageous for the analysis of powder samples with the device of the invention.

A third aspect of the invention refers to a device suitable for analysing a powder sample, the device comprising the insertion according to the previous description and the measuring cuvette detailed above.

In a preferred embodiment of the device:
- the device comprises a switchable locking system that is adapted to selectively open or block the outlet of the insertion; and/or
- the device comprises one or more thermohygrometers.

The switchable locking system may be manually controlled by the operator of the device or, alternatively, it can be remotely controlled by the computer means automatically, for example, by being triggered by an electronic signal. The switchable locking system may comprise an actuator or other hardware means in order to receive the electronic signal from the computer means and, depending on said signal, opening or closing the outlet of the insertion. Furthermore, the switchable locking system can be integrated into the measuring cuvette or cannot be part of said measuring cuvette. Regardless of which switchable locking system is chosen, it must be able to selectively open and close the outlet of the insertion.

In a more preferred embodiment of the device, it further comprises:
- a backlight source of illumination suitable for partially or totally illuminating the chambers through the base; and
- image acquisition means which are adapted to acquire one or more images and/or videos of the analytical chamber and/or the loading chamber and to send said images and/or videos to computer means; and
- the computer means configured to provide data acquisition and processing, said computer means controlling both the backlight source of illumination and the image acquisition means.

Preferably, the backlight illumination(s) is a uniform, uneven and/or scattered light emitting diode (LED) source (e.g., emitting white 6000 K light), an electroluminescence (EL) devices, and other LCD backlights (CCFL, EEFL, FFL, etc) or the like, which can comprise any wavelength of the spectrum. Such background illumination can be adjusted in intensity to detect underlying physical characteristics by transmitting light trough the base to the powder sample, e.g., changes in crystallinity and/or opacity, and to optimize image processing, e.g., particle edge detection. More preferably, the backlight illumination(s) cover both chambers of the measuring cuvette, including the insertion accommodated in the loading chamber.

In a particular embodiment of the device, the image acquisition means comprise at least one camera providing digital imaging and:
- the image acquisition means operate in ultraviolet, infra-red or visible ranges; and/or
- the at least one camera of the image acquisition means is monochrome; and/or
- the at least one camera of the image acquisition means is attached to one or more objectives or magnifying lenses, said at least one camera being configured to provide microscopic imaging; and/or
- the at least one camera of the image acquisition means comprises 2 or 3-axis positioning means.

The image acquisition means can operate by collecting images in different frequency bands (e.g., can comprise cameras operating in either ultraviolet, infra-red, or visible ranges). Preference is given to high resolution cameras (e.g., 20 megapixels) and more preferably to monochrome cameras that are known to provide a more accurate resolution of variations in light intensity passing through the base in grayscale. The image acquisition means may comprise at least one camera providing digital imaging, whether in real-time or not and, preferably, at least one of the cameras is used for microscopic imaging, e.g., by using objectives or magnifying lenses (from 2 to 100x). Preferably, at least one of said cameras comprises 2 or 3-axis positioning means for precise inspection of the powder sample during analysis with the device, i.e., the powder sample is imaged step by step, which in turn comprises computer and/or manually controlled means.

Furthermore, the image acquisition means can be implemented as one or more cameras that allow independent image acquisition of the analytical chamber and the loading chamber, or of both simultaneously. Image analysis techniques may be applied to process the collected images to obtain parameters or numerical descriptors of the flow properties of the powder sample. For example, area, dispersion, perimeter and uniformity in the powder flow pattern. Other example descriptors are the angle of impact after the powder sample hits at least one of the disturbance pins in the analysis chamber and/or the behaviour of the powder sample in the insertion after applying the moving force.

In another embodiment, the device is characterized in that:
- the size of the disturbance pins is from 2 to 8 mm in diameter in case that the disturbance pins exhibit a circular geometry and/or;
- the size of the disturbance pins is from 2 to 8 mm width in case that said disturbance pins exhibit a non-circular geometry; and/or
- at least one of the disturbance pins is located at a distance between 2 and 12 mm from the outlet of the insertion.

Throughout this document, a "circular geometry" refers to a volumetric body comprising a base with a circular or rounded shape, such a circle or an ellipse. For instance, a cylinder is considered a "circular geometry" within the scope of this specification. A "non-circular geometry" refers to those volumetric bodies whose base does not exhibit a rounder shape (e.g., other polygonal shapes like triangle, square, etc.). Furthermore, the one or more disturbance pins are devised in any geometry (size, shape, etc.). For instance, it can be rectangular, circular, triangular, or can comprise a semi-circular or a curved segment, etc.

In a preferred embodiment, the device further comprises a motion platform connected to the measuring cuvette, said motion platform being adapted to apply a moving force to the measuring cuvette.

The moving force moves the device embodiment from an initial or home position, where the measuring cuvette is placed in the motion platform of the device, to an end position where, preferably, image acquisition and lightning of the measuring cuvette occur. The moving force is preferentially applied at the home position and can vary in distance, speed and/or acceleration. Advantageously, an initial acceleration is applied to the measuring cuvette that is smaller than the subsequent deceleration to generate a flow of powder sample migrating from the loading chamber to the analytical chamber where it impacts the pin(s).

Preferably, the motion platform comprises a motor and a controller for said motor; and the moving force is defined in a horizontal direction determined by the main axis of the funnel portion of the insertion. The controller may comprise computing means or a programmable device (e.g., a PLC, an FPGA or a microcontroller board such as Arduino) that sends signals to specify the motion profile. Said motion platform may comprise at least one servomotor. Other embodiments of the mobile platform may comprise, for example, a stepper motor with vibration-free steel shafts.

Advantageously, to achieve an inch-perfect motion and precise synchronization of the image acquisition means, the motion platform can comprise an encoder or alternatively a potentiometer, resolver or a Hall effect transducer and/or one or more displacement sensors (e.g., laser, mechanic or magnetic sensors). In this way, displacement, velocity and accelerations of the motion platform can be precisely adjusted. In addition, the motion platform can reach accelerations of 4 G (g-force), which in turn promotes powder particles interactions when such particles are forced to flow through different geometries of the insertion. This is particularly useful for the analysis of cohesive materials to overcome prevailing cohesive forces or for larger particle size materials to be subjected to different shear strengths, thus describing the interaction forces, e.g., those associated with lubricant addition. In addition, different states of compaction can be achieved depending on the acceleration applied to the powder sample.

In a preferred embodiment of the device, it further comprises a feeding accessory made partially or totally of antistatic material, which comprises:
- a lower portion geometrically adapted to fit the loading chamber and/or the insertion;
- a locking mechanism;
- a central portion comprising an aperture which can be blocked by the locking mechanism and suitable for systematically releasing the powder sample to the lower portion; and
- an upper portion suitable for collecting the powder sample; the upper portion being adapted to be screwed into central portion and suitable for systematically depositing the powder sample to the closed aperture of the central portion;
- a cap adapted to be mechanically fixed to the upper portion, said cap being adapted to hold an interchangeable sieve such that said sieve is placed between the cap and the upper portion;
and wherein the locking mechanism is adapted to be allocated in the central portion.

The lower portion fits the loading chamber and/or the insertion so that the feeding accessory can always be placed in the same position by mechanical pressure. The loading accessory is preferably not fixed to the measuring cuvette and is partially or totally made of antistatic material for the above-mentioned advantages.

Advantageously, the powder sample is first passed through a sieve that normalized certain physical properties such as, for example, agglomerate-formation due to MgSt that can affect the charge of the powder sample into the feeding accessory and thus the reliability of the analysis. Said sieve, preferably made of stainless steel, is located in the upper part of the loading attachment and is easily interchangeable, so that the mesh aperture can be adjusted according to the physical properties of the powder sample. The powder sample is then collected in the narrow end of the funnel in the upper portion, where it is disposed until the locking mechanism is released. More advantageously, the loading accessory provides consistent discharge of the powder sample by gravity into the centre of the dome-shaped portion of the insertion. Additionally, the feeding accessory can be used to weight the powder sample.

In a particular embodiment, the device further comprises:
- at least a darkfield source of illumination, which in turn comprises tilt means controlled by the computer means and/or manually for adjusting the angle of the darkfield illumination source with regard to the base;
- a programmable device configured to switch between the backlight illumination source and the darkfield illumination source and/or a combination of both.

Advantageously, an arrangement with two or more darkfield and/or one or more brightfield sources provides photometric stereoscopic imaging and/or topographical analysis of the powder sample surface, i.e., observation of the powder sample using reflected light. Thus, the combination of darkfield and blacklight (transmitted light) illuminations in the invention further extends the powder characterization by connecting certain physical properties of the powder sample, such as particle size distribution (PSD) and morphology, to its processability. For instance, PSD uniformity and spherical particle deviations can greatly affect the packing density and powder flowability and must be controlled. Moreover, the backlight removes signal interference external to the powder flow pattern and provides additional valuable information that would not be obtained without the light transmitted through the sample, e.g., for detecting agglomerates, quantifying the ratio between spherical and irregular particles, the detection of small particles in the powder sample or clear visualisation of single particles. In particular, this combination is advantageous in the device of the invention as it can describe the depth/thickness of the layers forming the powder flow pattern, thus providing a three-dimensional representation of the powder flow pattern during image processing.

Preferably, the image processing is automated and calculates several PSD parameters, textual and graphical outputs. More preferably, the image acquisition and illumination of the measuring cuvette occur at the end position, i.e., when the powder sample has been transferred to the analytical chamber and is at rest. In this way, this static imaging provides high resolution for large particles and morphological analysis, which is specially recommended for pharmaceutical ingredients. Advantageously, this particular embodiment also offers versatility in that other sample states can be analysed, e.g., liquid suspensions, particles captured on filters, emulsions, thin powder layers or OSD forms.

In a preferred embodiment, the device further comprises vibration-generating means adapted to be applied to the measuring cuvette.

The possibility of varying the geometry of the insertion of the invention facilitates the dispersion of a small amount of powder sample. Also, the configuration of the movement in the device of the invention and the applied method promotes the dispersion of the powder sample, i.e., by limiting the interaction mechanisms between particles.

Advantageously, the vibration-generating means ensure consistent dispersion of the powder sample between measurements, i.e., separate the particles from each other, and are applied to the measuring cuvette; either before, during or after applying the moving force. Advantageously, these vibrations facilitate the analysis of cohesive powders with the device of the invention without damaging them by reducing the governing attractive interaction forces in said powders. For instance, the vibration-generating means can be part of the motion force applied to the motion platform by applying one or more bidirectional pulses of varying the speed and length or, alternatively, can come from different and/or external means to it, such as an acoustic or ultrasonic transducer, a vibration generating motor, an electromagnet or the like. Thus, the vibrations can be acoustic and/or mechanical. Optionally, the image acquisition means, e.g., the camera(s), are synchronized with the vibration generating means to provide a suitable image of the powder sample during or after applying the vibration.

In those embodiments of the device which do not comprise the vibration-generating means, the base area of the loading chamber is preferably made of a PFTE plate. Alternatively, in those embodiments of the device comprising the vibration-generating means, the base area of the loading chamber is preferably made of a material thinner than the PFTE. For instance, in such embodiments the base can be made of stainless steel plates or similar, which do not dampen the transmission of the vibrations to the powder sample.

A fourth aspect of the present invention refers to a method according to claim 14 for characterizing the particle surface characteristics of a powder sample.

The aforementioned method is suitable for both cohesive and non-cohesive powder samples. Throughout this document, the flow properties of medium-size microcrystalline cellulose (MCC PH102) are used as reference to define a material as "cohesive or non-cohesive", as this is recognized as the limit for acceptable flowability during tableting. Thus, a "cohesive or moderately cohesive" material is understood as a powder sample with poorer flow properties than MCC PH102, typically a mean particle size or Dv50 of less than 100 µm, while a "non-cohesive" material is understood as a material with better flow properties than MCC PH102, typically a mean particle size greater than 100 µm. However, other physicochemical properties of the materials, such as the number of fines or moisture content, may affect the interaction mechanisms between particles and therefore the above classification of materials is understood to as non-limiting when using the method and/or the device.

For instance, the moving force is related to the customized shear strength measurement, which can be outputted as numerical data by the device of the invention. When an accelerated linear motion causes a displacement of the particles (transferred momentum), a powder flow pattern is eventually produced after dissipation of the kinetic energy within the system. This momentum is lost as kinetic energy due to internal and wall frictions, in addition to subsequent impaction with the pin(s), which allows defining the residual kinetic energy after the powder sample flows through the outlet from the loading chamber to the analytical chamber in which the powder sample spreads. This new equilibrium corresponds to the magnitude of the interaction forces or resistance of the powder to such displacement (powder strength).

The consolidation step(s) comprises different degrees of compaction/packing of the powder sample to emphasise prevailing cohesive forces in cohesive materials. The packing density becomes particularly important for the analysis of cohesive or moderately cohesive materials, such as small-sized microcrystalline cellulose (MCC PH101), as these materials often exhibit highly variable densities that are largely dependent on the processing of the material, e.g., the addition of lubricant. Note that the consolidation stage requires that the outlet of the insert into the loading chamber is initially closed by the switchable locking system in the initial position. Thus, when the motion force is applied to the measuring cuvette of the device by the motion platform to generate a flow, the powder sample is prevented from passing through the outlet and is thus compacted in the narrowest part of the funnel portion of the insertion. After consolidation, the outlet is opened, and the movement force is applied again from the initial position so that the powder flow migrates into the analytical chamber. Preferably, the consolidation step(s) and the subsequent moving force can be adjusted separately.

Advantageously, a complete migration of the compacted powder sample into the analysis chamber after the consolidation step minimizes other interaction forces within the system that may impact the analysis, i.e., particle-particle and particle-insertion interactions. In this way, the powder strength can be described precisely, which in turn characterizes the MgSt distribution in the cohesive sample. This is achieved by reducing the sample size (e.g., 0.1 cm³ or 38 mg in the case of MCC PH101) and by measuring in a low shear flow regime (i.e., using insertions with geometries that avoid wall friction, such as those that have funnel portions with a smaller acceptance angle and/or larger outlet aperture). Hence, the consolidation step reduces the kinetic energy loss of the powder sample before passing through the outlet, resulting in larger powder flow patterns using less sample. More advantageously, the consolidation step provides unprecedented sensitivity of the method and/or device to subtle changes in the physical properties of MCC PH101. For instance, flow variations caused by the addition of 0.10% w/w of MgSt can be detected.

Alternatively, the consolidation step can be also applied to non-cohesive materials in order to compact and/or place the non-cohesive lubricated sample in the dome-portion of the insertion by applying a moving force from the end position to the home position (with the outlet of the insertion closed) or by applying a fast acceleration and slow deceleration motion profile from the home position to the end position that produces a similar compaction and/or migration of the lubricated sample in the dome-portion of the insertion. It should be emphasized that lubricant distribution is measured as a function of particle-to-particle contacts, so that larger non-cohesive materials whose particles increasingly behave as single units due to gravitational forces require to be subjected to low/moderate shear stresses that magnify the interactions. That is, insertions with a wider funnel portion acceptance angle and/or a smaller outlet opening are preferred for such materials.

More advantageously, the compressibility of a lubricated sample or any other material can be analysed according to the previous consolidation step and the compaction of the powder sample in the funnel portion of the insertion. Alternatively, other geometries of the measuring cuvette are also considered, for example, a receptacle shape or any other disposition that allows measurement of the ratio between the mass of the powder and the volume occupied by the powder after consolidation by the moving force. For instance, compressibility is very important aspect in powder technology, as the packing properties of a powder affect the processes, e.g., bulk storage and compaction.

In a further embodiment, the method of the invention comprises:
- operating vibration-generating means to induce a vibration in the measuring cuvette during the migration of the sample of lubricated powder from the loading chamber to the analytical chamber; and/or
- performing at least a standardization step by operating the vibration-generating means when the measuring cuvette is placed at the home and/or end positions.

The additional standardization step(s) at home position further standardizes the particle arrangement of the loaded powder sample in the measuring cuvette, which means increased repeatability. Alternatively, the standardization step(s) can be performed at the end position to get additional descriptors of the material flow properties during image processing. A comparison of measurements with and without such standardization can be considered, as it gives more information on the effect of the initial and/or final particle arrangement of the powder sample on the interaction forces, e.g., electrostatic forces. This standardization can be performed by the vibration-generating means already described. Furthermore, the image acquisition means can be adjusted to characterize the already described step(s) of standardization and consolidation. This includes, for instance, the acquisition of video or image sequences that define in real time (or not) the behaviour of the powder sample at the home or end positions.

Unless stated to the contrary, any use of the words such as "including", "containing", "comprising", "having" and the like, means "including without limitation" and shall not be construed to limit any general statement that it follows to the specific or similar items or matters immediately following it.

Most of the foregoing embodiments are not mutually exclusive but can be implemented in various combinations. As these and other variations and combinations of the features discussed above can be utilized without departing from the invention as set forth in the claims, the foregoing description of the embodiments should be taken as illustrative and not as a limitation of the invention as defined by the appended claims. In this way, features from different embodiments can be combined unless explicitly disclosed otherwise.

### DESCRIPTION OF THE DRAWINGS

These and other features and/or advantages of the invention are detailed hereinafter in the attached drawings, which depict a preferred but non-limiting example embodiment of the insertion, the measuring cuvette, the feeding accessory and the methods.
Figure 1 This figure displays different views of an embodiment of the insertion according to the present invention, which comprises a dome-shaped portion (halfcircumference section) and a funnel portion. Figure 1A is an isometric view of the insertion, Figure 1B is a top view of the insertion and Figure 1C is a bottom view thereof.
Figure 2 This figure illustrates different views of the base of the measuring cuvette, which further comprises the disturbance pin.
Figure 3 This figure illustrates different views of the lower part of the measuring cuvette which holds the base of Figure 2 and is coupled to the upper part of Figure 4.
Figure 4 This figure illustrates different views of the upper part of the measuring cuvette that is composed of two chambers, the loading chamber and the analytical chamber.
Figure 5 This figure illustrates the assembling of the lower and upper part of the measuring cuvette together with the base. Figures 5A and 5B show two views of the entire measuring cuvette. Figure 5C shows a top view of an embodiment of the measuring cuvette, where different materials are indicated with different colors.
Figure 6 This figure depicts a schematic representation of the device according to the present invention. The device comprises the measuring cuvette of Figures 2-5, which is illuminated by different illumination sources and fed by a feeding accessory. Image acquisition means are configured to capture images of the measuring cuvette and send said images to computer means.
Figure 7 This figure shows different perspectives of the lower portion of a feeding accessory suitable for its use with the device of the present invention. Figure 7A corresponds to a bottom view of said lower portion, while Figure 7B is a top view and Figure 7C is a side view thereof. Figure 7D illustrates an isometric view.
Figure 8 This figure illustrates different views of the central portion of a feeding accessory which is adapted to fit with the lower portion of Figure 7 and the upper portion of Figure 9. The following views are displayed: Figure 8A shows a side view, Figure 8B represents a bottom view and Figure 8C corresponds to an isometric view.
Figure 9 This figure depicts several views of the upper portion of the feeding accessory. Figure 9A shows a isometric view, Figure 9B represents a side view, Figure 9C is a bottom view and Figure 9D corresponds to a top view.
Figure 10 This figure illustrates the locking mechanism of the feeding accessory. The locking mechanism piece is adapted to be inserted in the central portion of Figure 10.
Figure 11 This figure illustrates the assembling of the feeding accessory portions of Figures 7-10.
Figure 12 This figure illustrates the switchable locking system attached to the insertion.
Figure 13 This figure illustrates the arrangement of the switchable locking system in the measurement cuvette.
Figure 14 This figure shows a flowchart with the basic steps of the method of the invention.
Figure 15 This figure shows a flowchart of the method of the invention similar to that of Figure 14 but comprising a step of operating vibration-generating means to induce a vibration in the measuring cuvette.
Figure 16 This figure summarizes the results of flowability and tensile strength for MCC PH101 cohesive mixture (2% MgSt), which is considered as a cohesive mixture.
Figure 17 This figure summarizes the results of flowability and tensile strength for MCC PH102 and pregelatinized starch (0.5% MgSt), which is considered as a non-cohesive mixture.
Figure 18 This figure summarizes the results of flowability and tensile strength for MCC PH102 and agglomerate lactose (0.5% MgSt), which is considered as a non-cohesive mixture.

### DETAILED DESCRIPTION OF THE INVENTION

In the following, preferred embodiments of the insertion, device and method according to the present invention are described. Note that these non-limitative realizations are just examples and that other embodiments are included in the scope of the invention, solely limited by the attached set of claims.

### Insertion (1) of the invention

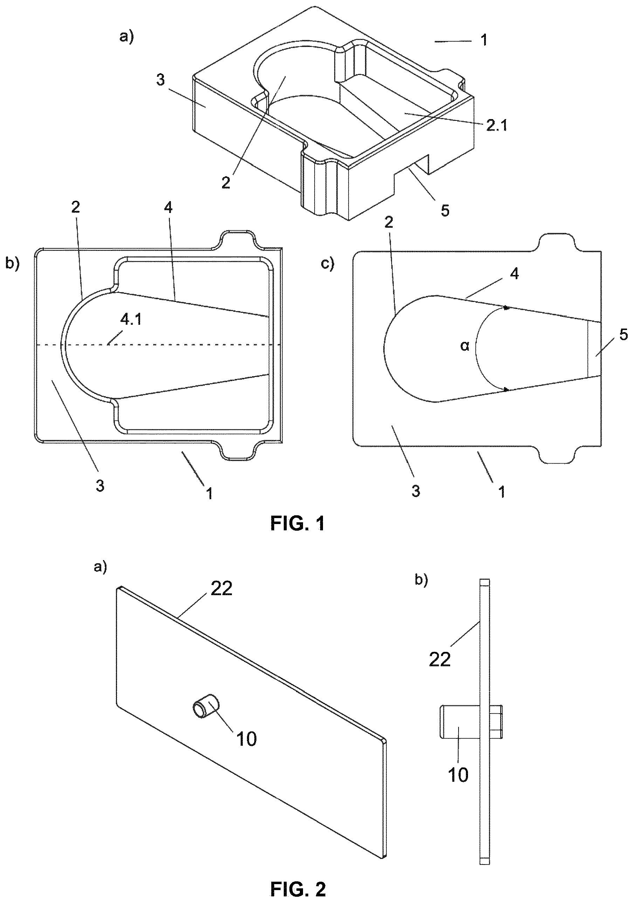

Figures 1A, 1B and 1C display different views of a preferred embodiment of the insertion (1) according to the present invention. The insertion (1) comprises:
- a dome-shaped portion (2), which is configured to receive a powder sample through an inlet (2.1);
- a funnel portion (4) connected to the dome-shaped portion (2) and in flow communication with said dome-shaped portion (2), said funnel portion (4) comprising an outlet (5) in the narrowest part of the funnel portion (4), said outlet (5) configured to feed an analytical chamber (9) with a powder sample; and
- a frame (3) surrounding the dome-shaped portion (2) and the funnel portion (4), the frame (3) being adapted to be the core of the structure of the insertion (1). The frame (3) can be manufactured in any desired geometry. For instance, in Figure 1, the frame (3) is a stepped structure to facilitate the introduction of a powder sample into the dome-shaped portion (2).

The frame (3) is adapted to fit into a fixed portion (8) of a measurement cuvette (13) of the device (12) detailed below.

Particularly, the insertion (1) comprises 316L stainless steel, the funnel portion (4) defines the acceptance angle (α) of 22.5° (see Figure 1), where this angle is defined with respect to the outlet (5) and the diameter of the dome-shaped portion (2) which is 10 mm; and the length of the funnel portion (4) is 22.5 mm. The larger acceptance angles apply to non-cohesive powder samples because the funnel portion (4) promotes the particle-to-particle contacts prior to and during flow through the insertion outlet (5). Also, the main axis (4.1) of the funnel portion (4) defines the so-call horizontal direction.

The insertion (1) of the invention is preferably detachable, so it can be attached and separated from the device (12) of the invention when needed. In this way, depending on the type of powder sample to be analyzed, the insertion (1) can be replaced in the device.

### Measuring cuvette (13) and device (12) of the invention

This device (12) is suitable for analysing a powder sample and comprises a insertion (1) and a measuring cuvette (13). The base (22) is made of a translucent material and is configured to receive illumination from at least one illumination source (14, 19) and to allow said illumination to pass through the base (22). The base is the main structural element of the device (12). Preferably, the base (22) is partially made of polytetrafluoroethylene, PTFE; and at least the surrounding embodiment of said base (22) comprises an antistatic material. The base (22) is displayed in Figure 2 and consists of a flat plate comprising a single disturbance pin (10).

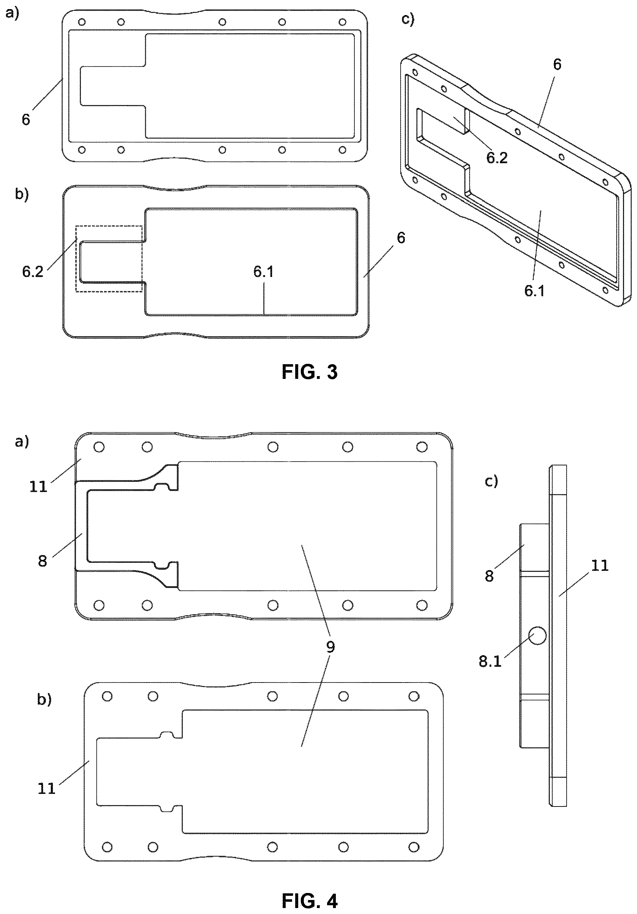

In this particular embodiment, the measuring cuvette (13) has been manufactured by assembling two different parts (6, 11):
a) A lower part (6) of the measuring cuvette (13), shown in Figure 3, which is designed to be mounted on the base (22). The lower part (6) comprises a first portion (6.1) for housing an analytical chamber (9) and a second portion (6.2) for housing a loading chamber (7).
b) An upper part (11) of the measuring cuvette (13), displayed in Figure 4, which is adapted to be mounted on the lower part (6). The upper part (11) comprises the analytical chamber (9) and a fixed portion (8). The fixed portion (8) is shaped to house the insertion (1), which in turn can be connected to a feeding accessory (18), explained bellow, thus allowing the entry of a powder sample into the device (12) through the inlet (2.1), or recess, of the insertion (1). In this way, the fixed portion (8) together with the insertion (1) constitute the loading chamber (7) such that the insertion (1) is substantially complementary and fits into the fixed portion (8).

The two parts (6, 11) of the measuring cuvette (13) accommodate the base (22).

As illustrated in Figure 4, the analytical chamber (9) is located next to the loading chamber (7) and in flow communication with said loading chamber (7). The disturbance pin (10) is made of 316L stainless steel and exhibits a cylindrical shape with a diameter of 5 mm.

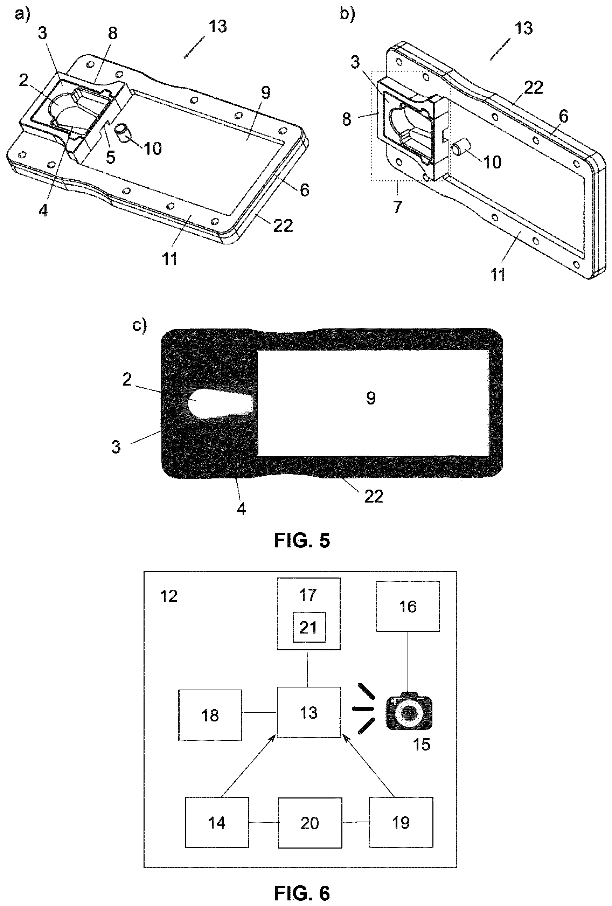

Figure 5 shows the assembly of the base (22) and the parts (6, 11) of the measuring cuvette (13). The disturbance pin (10) is placed next to the outlet (5) of the insertion (1). In this way, the powder sample passing through the outlet (5) collides with the disturbance pin (10) as it spreads into the analytical chamber (9). The loading chamber (7) and the analytical chamber (9) are connected through an outlet (5) of rectangular shape 5 mm in length. The device (12) further comprises a switchable locking system (23) that is adapted to selectively open or block the outlet (5), as later explained. Advantageously, the size and the shape of the outlet (5) and the incidence angles with respect to the funnel portion (4) of the insertion (1) are customized to modify the powder strength or forces restricting the flow of the powder sample. For instance, since lubricant distribution is measured according to particle-to-particle contacts, using different outlets sizes induces varying shear strength regimes, allowing effective characterization of lubricant distribution in powder samples with different flow properties, i.e., cohesive and non-cohesive materials.

In the embodiment shown in Figure 5, the antistatic material selected for the parts (6, 11) of the measuring cuvette (13) is POM, which is particularly advantageous for the following reasons:
- It withstands conditions where dust, powder or static electricity can cause problems.
- It exhibits a high electrical insulation and good dielectric properties prevent electrostatic charge accumulation. In particular, it prevents the accumulation or transfer of electrostatic charges to the insertion (1), since it insulates it from contact with other conductive materials.
- It has a unique combination of thermal, mechanical and chemical resistance properties to withstand sanitation methods.
- It has excellent anti-slip properties that prevent adhesion in case of contact of powder samples with the walls of different parts of the device (analytical chamber (9) walls, etc.).
- It provides high resistance to impact, wear and abrasion ensure long-lasting use.
- It presents a very low water absorption, which makes it ideal for cleaning between measurements without affecting the powder sample under test.
- Excellent machining required for its production.
- FDA grades approved for use in the pharmaceutical, food, etc. industry.

In addition, in Figure 5, PTFE is used as the translucent material of the base (22) as it provides the following features:
- It has a very low coefficient of friction, as well as strong anti-adherent properties, which minimizes the frictional forces of the powder sample with the base (22) and significantly increases the reproducibility and/or analytical sensitivity of the device (12).
- It acts as an electrical insulator (extremely low dielectric constant of 2.0), thereby preventing the accumulation of tribostatic charges (generated by the movement or collision of particles) that may generate repulsion, especially in small amounts of powder sample. Furthermore, it prevents the accumulation or transfer of electrostatic charges to the disturbance pin (10), since it insulates it from contact with other conductive materials.
- It acts as a diffuser for the backlight illumination source (14), so that the measurement area emits light uniformly (omnidirectionally).
- It presents a very low water absorption, which makes it ideal for cleaning between measurements without affecting the powder sample under test.
- It offers exceptional chemical resistance to sanitize pharmaceutical products (e.g. acetone) and has a high temperature tolerance to withstand heat-based sanitation methods (exhibits high thermal stability without obvious degradation below 440 °C).
- FDA approved for use in the pharmaceutical, food, etc. industry.

Figure 5C displays an embodiment of the measuring cuvette (13) where the different materials thereof are indicated by distinct colours:
- The white regions correspond to those of PTFE. These regions comprise the base of the dome-shaped portion (2) and the funnel portion (4) of the insertion, along with the base of the analytical chamber (9). In this way, this design benefits from the anti-adherent properties of PTFE during the flow of the powder sample in the device (12).
- The light gray regions, in particular the frame (3) of the insertion (1), are made of stainless steel (and more preferably 316L stainless steel for the advantages described above).
- The dark gray regions correspond to the antistatic material of the parts (6, 11), particularly POM.

Advantageously, the contrast between the black colour of the POM polymer and the opalescent white of the PFTE facilitates the imaging of the powder sample.

A preferred embodiment of the device (12) of the invention is displayed in Figure 6. In addition to the measuring cuvette (13) detailed above, the device (12) also comprises the following components:
- A backlight illumination source (14), in particular a white LED source, which provides illumination to the analytical and loading chambers (7, 9) of the measuring cuvette (13) through the base (22). For this reason, the base (22) is made of a translucent material that sharply defines the powder sample on the white background of the PFTE.
- The image acquisition means (15) are configured to acquire one or more images from the analytical and loading chambers (7, 9) and to send said images to computer means (16).
- The computer means (16) are configured to provide data acquisition and processing, with said computer means (16) controlling both the backlight illumination source (14) and the image acquisition means (15). Once the image acquisition means (15) acquire information from the powder sample flow is migrating from the loading chamber (7) to the analytical chamber (9), it is processed by the computer means (16). Optionally, the computer means (16) can acquire and analyse information from other sensors of the device (12), for instance, humidity or temperature sensors.
- A motion platform (17) connected to the measuring cuvette (13). During the measurement of a powder sample, the entire measuring cuvette (13) is subjected to a moving force linked to a specific motion profile applied by means of the motion platform (17). For instance, this motion profile comprise a slow acceleration followed by a rapid deceleration, and it generates a flow of the powder sample. The moving force is preferably applied in a horizontal direction, defined by the main axis (4.1) of the funnel portion (4). In this way, and according to Newtonian mechanics, the powder sample moves from the loading chamber (7) to the analytical chamber (9) through the outlet (5). In this embodiment, the motion platform (17) comprises vibration generating means (21) adapted to selectively apply a vibration pattern to the measuring cuvette (13).

Furthermore, as illustrated in Figure 6, the device (12) further comprises a feeding accessory (18) which is not attached to the loading chamber (7), wherein the feeding accessory (18) deposits the powder sample into the loading chamber (7) through the inlet of the insertion (1). Additionally, according to Figure 6, the device (12) further comprises:
- a darkfield illumination source (19), which in turn comprises tilting means controlled by the computer means (16) that adjust the angle of the darkfield illumination source (19) with respect to the base (22);
- a programmable device (20) configured to selectively switch between the backlight illumination source (14) and the darkfield illumination source (19) and/or the combination of both.

The measuring cuvette (13) is analysed in a compartment protected from the external illumination in a manner that prevents contamination or environmental exposure. Thus, the measuring cuvette (13) must be isolated to the greatest extent from external illumination and maximizing illumination from one or more backlighting illumination sources (14) and/or one or more darkfield illumination sources (19). Note that the operation of this device (12) relies on the fact that the image acquisition means (15) are correlated with the flow of the powder sample and, more importantly, with certain physical characteristics of the powder sample such as texture or shape of the particles. For this reason, external illumination disturbances must be avoided to achieve maximum image resolution.

### Feeding accessory (18)

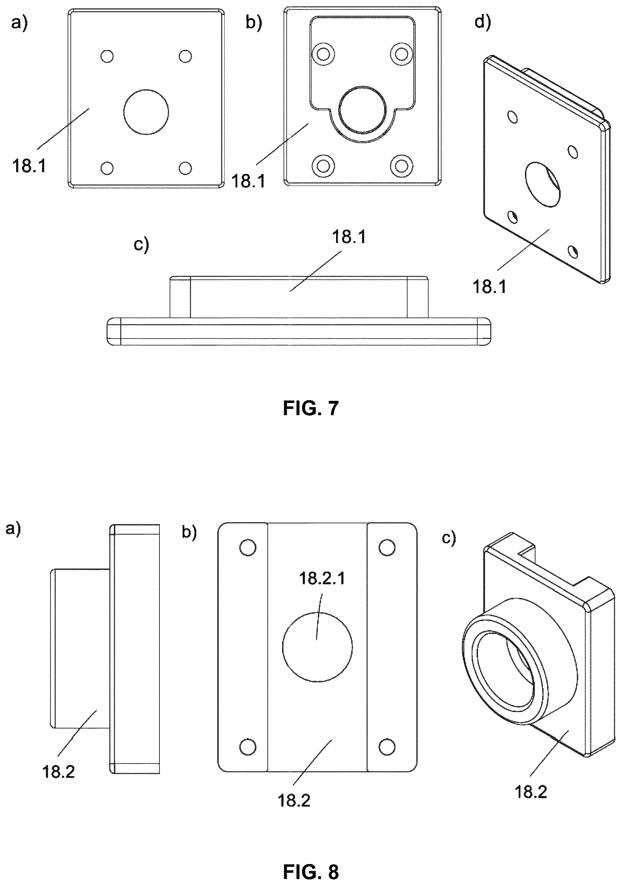

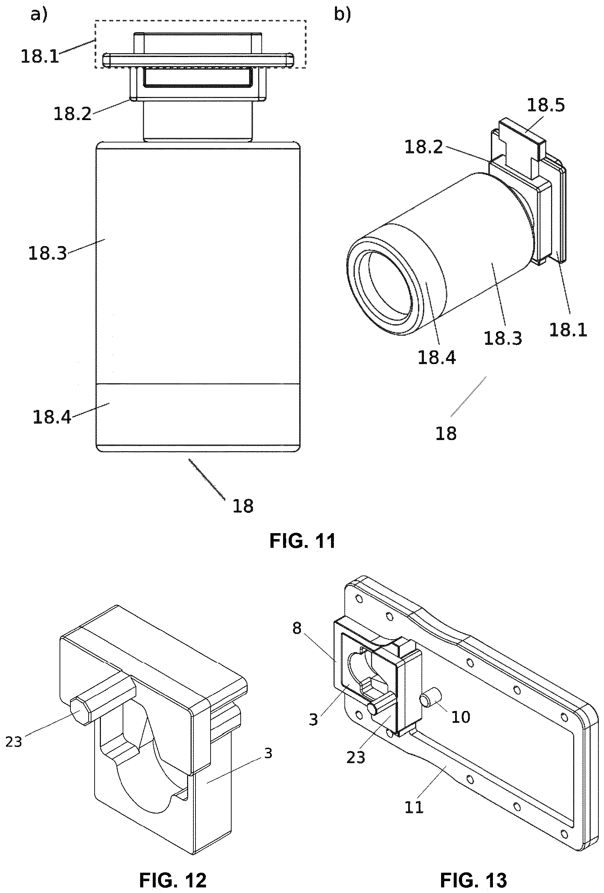

Figures 7-11, which are described as follows, contain different pieces (18.1, 18.2, 18.3, 18.4, 18.5) of a possible embodiment of the feeding accessory (18) suitable for feeding the device (12) of the invention. Then, all these pieces (18.1, 18.2, 18.3, 18.4, 18.5) can be assembled as shown in Figure 11. The feeding accessory (18) is partially or totally made of antistatic material such as graphite-containing polyethylene or any other material offering similar mechanical strength and antistatic properties.

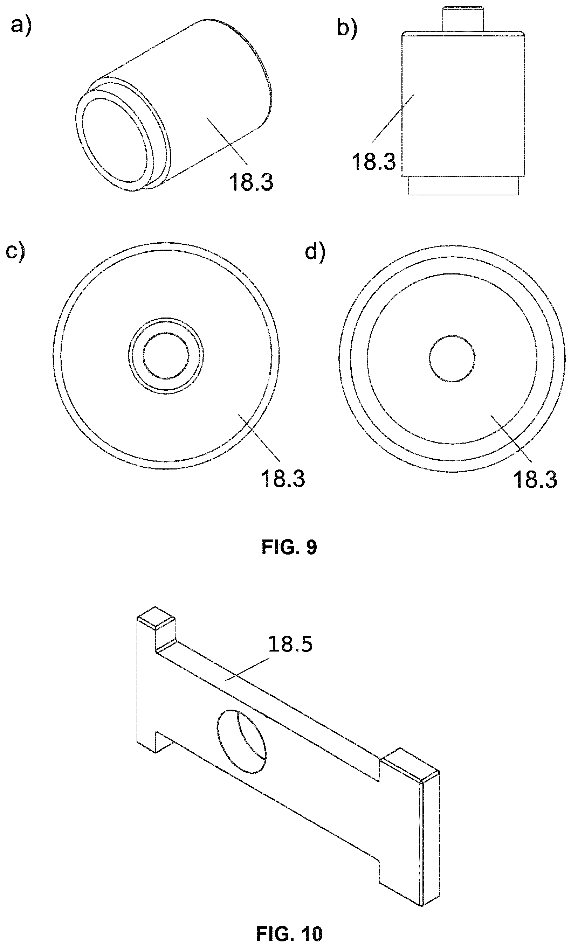

Figure 7 shows different perspectives of a lower portion (18.1) of the feeding accessory (18). Figure 8 illustrates different views of a central portion (18.2) which comprises an aperture (18.2.1) and it is adapted to be screwed to the lower portion (18.1) of Figure 7. Figure 9 comprises several views of an upper portion (18.3) of the feeding accessory (18). The upper portion (18.3) consists of a feeding funnel adapted to be placed into the joining piece (18.2) of Figure 8. Furthermore, Figure 10 shows a cap (18.4) of the feeding accessory (18) which is adapted to be mechanically attached to the upper portion (18.3) displayed in Figure 9. Said cap (18.4) is adapted to hold an interchangeable sieve (not shown in Figures) on the upper portion (18.3) of the feeding accessory (18).

Between the cap (18.4) and the upper portion (18.3), the sieve is placed. This stainless steel sieve screens the powder sample when it is introduced into the feeding accessory (18). Figure 10 illustrates the locking mechanism (18.5) of the feeding accessory (18). The locking mechanism (18.5) is adapted to be positioned between the lower and central portions (18.1, 18.2) and serves to selectively block the aperture (18.2.1). Figure 11 shows the whole assembling of the different pieces (18.1, 18.2, 18.3, 18.4, 18.5) to build the feeding accessory (18).

The feeding accessory (18), which has been already introduced in Figure 6, is substantially adapted to the shape of the fixed portion (8). Note that the inlet (2.1) of the insertion (1) is a recess adapted as the female connector of a tongue and groove arrangement; therein the lower portion (18.1) of the feeding accessory (18) is positioned. Moreover, the fixed portion (8) comprises a threaded hole (8.1) which secures the insert (8) to the measuring cuvette (13).

Figures 12-13 show a particular embodiment of a switchable locking system (23) that is placed in the insertion (1) and which constitutes a step of a method (100) for compacting the powder sample prior to measurement with the device (12). **In** this particular embodiment, the switchable locking system (23) is controlled manually to open or close the outlet (5) of the insertion (1). Preferably, the switchable locking system (23) is detachable so that it can be removed before measuring with the device (12).

### Method (100) of the invention

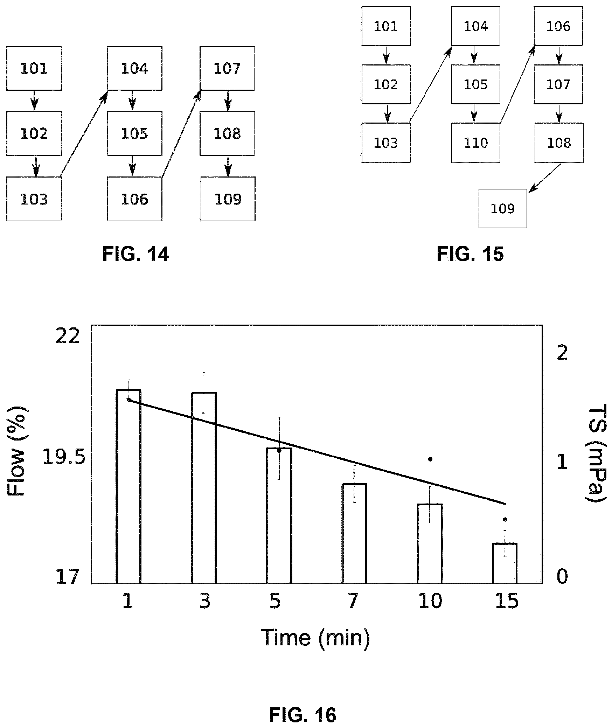

The invention further comprises a method (100) summarized in Figure 14, said method (100) being suitable for characterizing a lubricated powder sample. Said method (100) comprises the realization of the following steps using the device (12):
- selecting (101) a preferred insertion (1) and placing it in the measuring cuvette (13);
- optionally, cleaning (102) the measuring cuvette (13);
- depositing (103) the sample of lubricated powder into the loading chamber (7) through the insertion inlet (2.1) by using the feeding accessory (18);
- performing at least a consolidation step (104) by operating the vibration-generating means, the consolidation step (104) comprising compacting the lubricated powder sample before the migration of said sample from the loading chamber (7) to the analytical chamber (9);
- applying (105) a moving force to the measuring cuvette (13) of the device (12) by means of the motion platform (17) to generate a flow of the sample of lubricated powder that migrates from the loading chamber (7) to the analytical chamber (9); and the moving force displaces the measuring cuvette (13) from the home position to an end position;
- when the measuring cuvette (13) reaches the end position, illuminating (106) the measuring cuvette (13) of the device (12) by means of one or more sources of illumination (14, 19);
- acquiring (107) one or more images and/or videos of the flow pattern of the sample of lubricated powder at the end position by using image acquisition means (15);
- processing (108) the one or more images and/or videos by means of computer means (16) to obtain one or more parameters characterizing the sample of lubricated powder; and
- output (109) of the one or more parameters.

The moving force moves the measuring cuvette from a home (initial) position to an end position (i.e., in the image acquisition area, where images and/or videos of the analytical and/or loading chambers are collected).

Preferably, cleaning (102) the device (12) is carried out as follows; **It** is convenient to rinse the measuring cuvette (13) and either the upper part, including the cap (18.3, 18.4), or the entire feeding accessory (18) with water and/or another solvent (e.g., ethanol or isopropanol) to remove detrimental electrical charges and to avoid cross contamination. Furthermore, dried pressured air, nitrogen (N₂) and/or other gases, depending on the sample properties, remove the water molecules that may affect the analysis. Preferably, powder samples containing a high number of fines such as dry powder inhaler formulations may be dried with N₂ to minimise water adsorption on the powder sample.

The at least one consolidation step (104) of the lubricated powder sample involves compacting it in the insertion (1), to emphasise cohesion phenomena by producing a repeatable initial arrangement before the migration of said sample from the loading chamber (7) to the analytical chamber (9). This consolidation step (104) is performed by executing a stroke, in which the moving force is applied to the loading chamber (7) while keeping the outlet (5) blocked. The consolidation step (104) is particularly effective for measuring minor flow changes in the powder sample, such as those resulting from the physical properties of the lubricant, and further reduces the sample size (0.1cm³).

It is worth underlining the effect of mechanical energy input and its distribution during powder sample mobilization. As the acceptance angle α of the funnel portion (4) defined in the loading chamber (7) increases the outlet (5) size diminishes, and an increasing amount of the mechanical energy is lost as kinetic energy from internal friction and friction against the device (12) walls (e.g., the walls of the insertion (1), in addition to subsequent impaction with the disturbance pin (10). In contrast, when packing the powder sample right in the narrowest part of the funnel portion (4) during the consolidation step (104), which is characterised by a low shear regime (obtained by using wider diameter outlet (5)), the total energy loss within the system prior to flowing through the outlet (5) decreases, transferring more mechanical energy to the powder sample upon impact with the pin (10) and better defining the dominant intermolecular forces in more cohesive powder samples. For this reason, the consolidation step (104) plays an essential role in the characterization of cohesive or moderately cohesive materials. For instance, the consolidation step can be particularly important to characterize dry powder inhaler formulations.

Advantageously, the device (12) and method (100) of the invention allow monitoring of the lubricant blending process, which may speed up drug development and minimize rejects, as these are the first device (12) and method (100) to describe the lubricant distribution from powder samples or intermediate products.

Figure 15 corresponds to a variant of the method of Figure 14, but further comprising a new step: operating (110) vibration-generating means to induce a vibration in the measuring cuvette during the migration of the sample of lubricated powder from the loading chamber (7) to the analytical chamber (9).

Finally, it should be noted that the method (100) of the invention is not limited to the evaluation of the lubrication process, but is also suitable for characterising other processes or applications not studied so far where particle-level behaviour dominates over bulk behaviour. The method (100) of the invention may facilitate identifying and understanding the challenging cohesion phenomena that dominate many industrial processes and applications, e.g., dry powder inhalations.

### Experimental validation of the device (12) and the method (100)

In the following, the insertion (1), device (12) and method (100) of this invention are tested to describe the endpoint of the MgSt blending process. Dry powder coating with MgSt leads to a variety of physicochemical changes in the formulation and can adversely affect the final product. This modified particle-to-particle contact is correlated at different manufacturing stages: from customize flow shear strength measurements in intermediate products after blending using the invention to the effective bonding area during compaction or TS as a quality attribute of the tablet.

### a) Materials:

Model materials include different mechanical properties in which the softening effects from alkaline stearate lubricant MgSt are present. Furthermore, differences in powder flow properties depend on the physical properties of materials, both cohesive and non-cohesive materials are used. Magnesium stearate (MgSt) is studied as the most common lubricant agent in pharmaceutical development. Under Scanning Electron Microscope (SEM) imaging, MgSt appears as small, irregular-edged and lamellar aggregates.
- Sample 1 (cohesive material):

Fine grade microcrystalline cellulose MCC PH101 appears as a fine powder with a mean particle size Dv50= 63.2 µm (SEM imaging and laser diffractometry) and plasticdeforming mechanical properties. Thus, the tablet TS largely depends on the lubricant coting after blending. MCC PH101 is used to investigate the effect of MgSt in cohesive samples.
- Sample 2 (non-cohesive material):

MCC PH102 binary mixtures with DC lactose (Tablettose 100) and DC starch (Lycatab C) included viscoelastic and fragmenting behaviour to the cellulose plasticity, respectively. Therefore, MCC102 binary mixtures are used to investigate the effect of different mechanical properties that may be encountered during tablet manufacture. Starch appears as a moderately coarse white powder with a Dv50 of 100µm and lactose as a coarse white powder with a Dv50 of 200µm. MCC PH102 (Dv50= 102.6 µm) is considered as the boundary between an acceptable and a poor flowing excipient for high-speed compression. Both binary mixtures exhibit good-flowing properties.

### b) Powder flow characterization:

Shear strength is measured by using the method (100), which involves an image-based technology of miniaturized powder flow through the outlet/orifice (5). The method (100) examines a particulate powder material from an initial static equilibrium between the forces responsible for promoting powder flow (gravitational force, particle mass and/or external mechanical force) and the counteracting cohesive forces. A momentary accelerated linear motion then causes particles to move (transferred momentum) and produces a powder flow pattern at the new equilibrium that corresponds to the magnitude of cohesion or resistance of the powder to such displacement (powder strength). Powder flow is defined as the percentage of the analytical chamber (9) area (cm²) covered by powder material once it has crossed the outlet (5). Testing time and sample mass required are 5 minutes and 0.1-0.2cm³, respectively.

The measuring system is calibrated according to the lowest concentration of lubricant added (0.10% for MCC101 and 0.25% w/w MgSt for the DC mixtures). This provides the opportunity to adjust the transferred momentum and thus maximize powder strength measurement, depending on the physical properties of the materials.

### c) Compression studies and tablet physical properties:

Tablet TS is selected as secondary descriptor of the lubricant distribution to validate the invention. Small-scale tablet production is evaluated using a rotary tablet press (AM/13 Type, Ronchi, Milano, Italy) equipped with 2 flat punches with a straight secant line (diameter (Ø): 9 mm) at a defined speed of 36 rpm. Upper compression force and tablet weight remain constant for each material and are determined from the unlubricated blends (Target tablet weight: 250mg). For this purpose, the die-cavity volume is adjusted by setting the height of the lower punches before each compression cycle. TS values between 2 and 3 (Initial crushing strength (CS): 125±5N) are considered for unlubricated materials. Upper compression force values for each unlubricated blend as follows; MCC101: 2000N; DC-starch: 4000N; DC-lactose: 5500N. Increasing amounts of lubricant are pressed and 100 tablets (50 per punch) are rejected at the beginning of each batch to ensure uniform lubrication at the powder-tool interfaces, which has been recognized as the primary mechanism for reducing ejection force. Compression data is then collected from 50 tablets and the TS of 10 pcs was determined after 5 days of storage/relaxation. In the case of MCC101, considering the inconsistent filling of the press die that was especially present at high lubricant concentrations or long blending times, only tablets approaching the target weight are selected. TS is calculated from the diametrical breaking force and tablet dimensions using Fell and Newton equation. CS and tablet dimensions are measured using a Tablet Hardness Tester TBH 125 (Erweka Apparatebau GmbH, Germany) and a U30-F Digital Indicator (Sony Magnescale Inc, Japan).

### d) Measurement setup configuration for each sample:

### - Sample 1 (cohesive material):

At certain particle sizes (Dv50<100 µm) inter-particulate weak polarizing vdW forces begin to overcome gravity dominating powder behavior. When these attractive forces exceed the particle weight by at least an order of magnitude, the powder becomes very cohesive, and the particles no longer flow individually but as aggregates. Moderately cohesive materials such as MCC PH101 often exhibit highly variable densities (compressibility) that depended to a large extent on the material processing, for example, the lubricant addition. The packing density of the material then defines the number of contacts between particles and thus powder cohesion. Therefore, a consolidation step (104) is applied to produce a repeatable initial arrangement of the powder sample. This, in addition to a reduced sample size (0.1 cm³) and a low shear regime (wide and rectangular outlet (5) with 5 mm length) that prevents from other interaction forces within the system such as wall friction, is particularly effective for measuring minor flow changes depending on the physical properties of the lubricant. The consolidation is performed by executing a stroke with the outlet (5) of the device (12) blocked by means of the locking mechanism (18.5). The acceleration is set at 2m/s².

### - Sample 2 (non-cohesive material):

Conversely, at certain particle sizes (Dv50> 100 µm) gravity begin to overcome the magnitude of inter-particulate forces. Since lubricant distribution is measured according to particle-to-particle contacts, larger particle sizes such as DC mixtures that increasingly behave as single units are subjected to low/moderate shear. Thereby, a sample size of 0.2cm³ and different shear rates are studied. As the angle of the funnel portion (4) decreases according to the outlet size (5), an increasing amount of kinetic energy is lost from internal (and wall) friction in addition to subsequent impact with the disturbance pin (10). To achieve good sensitivity at 0.25% w/w MgSt, the outlet (5) size is smaller than in the case of the cohesive material (in this case, outlet (5) with 3 mm length) and the DC lactose mixture is measured at a lower acceleration (1.7 m/s²). Otherwise, increased interparticle dynamic friction appeared and this larger particle size material does not flow steadily through the outlet (5) of the device (12) during the measurement.

### e) TS and flow measurements for cohesive mixture:

The lubricant distribution determined the bonding properties of the blend and cohesion phenomena, thus affecting both TS and powder strength of fine grade MCC101. This includes degree of delamination, the penetration into the surface roughness and the extent of the stratified lamellar coating afterwards. A concentration of 0.5% MgSt is selected to show the relationship between variables, which corresponds to one of the lowest, and therefore most challenging to characterize, concentrations used in pharmaceutical development

Figure 16 shows the flowability (represented as a bar plot) and TS (represented as dots) for MCC PH 101 (0.5% MgSt). A positive correlation defines the relationship between both variables (r: +0.763).

### f) TS and flow measurements for non-cohesive mixture:

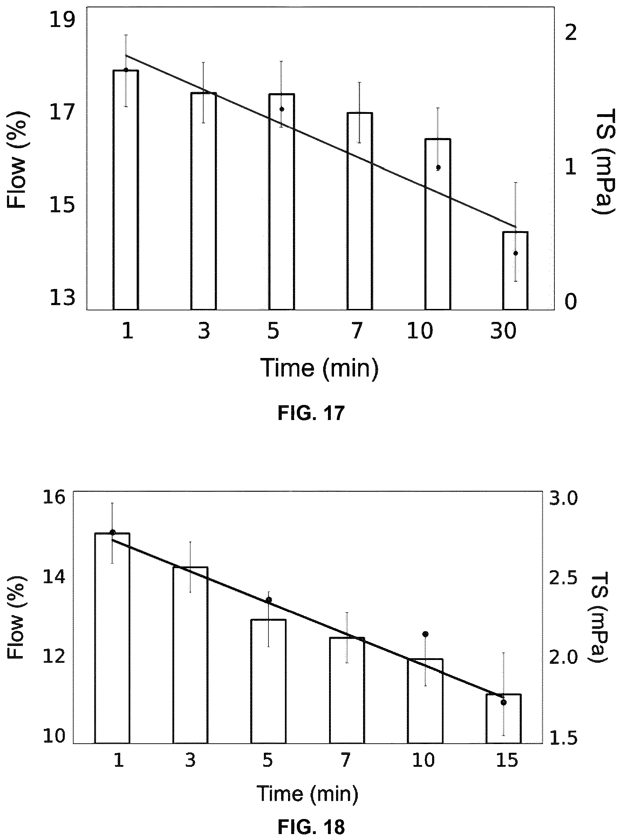

Figures 17-18 shows the flowability (represented as a bar plot) and TS (represented as a line) for DC binary mixtures (0.5% MgSt). For this non-cohesive materials, the surface of substrate particles is also gradually coated with delaminated MgSt over time, decreasing either the effective bonding area or lubricant-free area (TS decreases) and powder strength or flow values. Flow and TS shows a strong correlation (r: +0.800 and +0.826 for DC-starch and DC-lactose, respectively).

Flow data described the MgSt coating after blending and so depended on the substrate physical properties while TS resulted from the mechanical properties during compaction. As observed in Figure 17, customized Flow measurements described the extent of MgSt for both formulations, regardless of product morphology. The higher shear applied to DC-lactose (Figure 17) resulted in lower overall Flow values compared to DC-starch (Figure 18). **In** contrast, DC-starch has significantly reduced TS while DC-lactose is less sensitive to the lubricant coating during compaction and demonstrates good tabletability at constant compression force. This strong variability associated with the formulation determined the relationship between both descriptors as product-specific.

### Conclusions

Dry coating with MgSt leads to a variety of physical changes in the sample formulation. The device (12) and method (100) of the invention are appropriate to describe the non-uniform distribution of MgSt in the intermediate powder products. A customized measurement setup for each formulation, depending on the material physical properties, predicts the impact of blending time in the tensile strength (TS) as a quality attribute of the tablet. When dealing with cohesive material, the consolidation step (111) increases the reliability of the measurements obtained with the device (12). On the other hand, when the sample is a non-cohesive material, it is sufficient (although consolidation can also be applied as mentioned above) to study different angles of incidence provided by the funnel portion (4) in the insertion (1) which allows studying different shear rates or magnitude of contact between particles. That is, the invention determines the end point for the lubricant blending unit operation according to the modified behaviour at interfaces or distribution of MgSt in the particle surface. High linearity between both descriptors was found for materials with different physical and mechanical properties, which is defined as product-specific. Advantageously, the method is nondestructive and effective at short blending times and small amounts of lubricant, requiring few experiments and a small amount of sample to characterize the lubricant blending unit operation.

## Claims

1. A detachable insertion (1) for cooperating with a measuring cuvette (13), the measuring cuvette (13) being suitable for receiving a powder sample, and the insertion (1) comprising:
- a dome-shaped portion (2), which is configured to receive the powder sample through an inlet (2.1);
- a funnel portion (4) connected to the dome-shaped portion (2) and in flow communication with said dome-shaped portion (2), the funnel portion (4) comprising an outlet (5) placed at a narrowest part of the funnel portion (4), the outlet (5) being configured to feed an analytical chamber (9) of said cuvette with the powder sample; and
- a frame (3) surrounding and integrating the dome-shaped portion (2) and the funnel portion (4);
wherein the insertion (1) comprises an inert material.

2. The insertion (1) of the previous claim, wherein:
- the funnel portion (4) defines an acceptance angle between 15-50°; and/or
- the shape of the dome-shaped portion (2) substantially exhibits a semicircle or comprises at least one curved segment; and/or
- the diameter of the dome-shaped portion (2) is within the range of 8-18 mm or, in case it comprises a curve or a curved segment, the chord of said curve or segment is within the range of 8-18 mm; and/or
- the length of the funnel portion (4) is within the range of 10-35 mm; and/or
- the outlet (5) substantially exhibits a quadrilateral shape, circular shape or a combination thereof.

3. Measuring cuvette (13) for cooperating with the detachable insertion (1) of the previous claims 1-2, the measuring cuvette (13) being suitable for receiving a powder sample, the measuring cuvette (13) being made of inert materials, wherein the measuring cuvette (13) comprises a base (22) and a set of two parts (6,11), comprising an upper part (11) and a lower part (6), the parts (6, 11) being joined together and containing the base (22); wherein the base (22) is made of a translucent material and adapted to accommodate the powder sample; and the base (22) is configured to receive lightning from at least one illumination source (14, 19) and to allow such lightning to pass through the base (22);
and wherein the upper part (11) comprises a loading chamber (7) and an analytical chamber (9); and wherein the lower part (6) holds the base (22) while enabling its illumination by means of the at least one source of illumination (14, 19); and wherein:
- the loading chamber (7) comprises a fixed portion (8) adapted to accommodate the insertion (1) according to any of the previous claims 1-2, such that the insertion (1) is substantially complementary and fits into the fixed portion (8); and
- the analytical chamber (9) is located adjacent to the loading chamber (7) and in flow communication with said loading chamber (7) through the outlet (5) of the insertion (1), and wherein the analytical chamber (9) comprises one or more disturbance pins (10).

4. The measuring cuvette (13) according to the previous claim 3, where:
- the measuring cuvette (13) and the base (22) are made of antistatic materials; and/or
- at least one of the disturbance pins (10) is cylindrical with a base diameter between 2 and 8 mm, and said at least one of the disturbance pins (10) is made of stainless steel and is adapted to be aligned with the outlet (5) of the insertion (1) and the funnel portion (4).

5. The measuring cuvette (13) according to any of previous claims 3-4, wherein the base (6, 11, 22) comprises a plate of non-sticky translucent material, preferably a fluoropolymer such as polytetrafluoroethylene.

6. A device (12) suitable for analysing a powder sample, the device (12) comprising the insertion (1) according to the previous claims 1 or 2 and the measuring cuvette (13) according to any of the previous claims 3-5.

7. The device (12) according to the previous claim, wherein:
- the device (12) further comprises a switchable locking system (23) that is adapted to selectively open or block the outlet (5) of the insertion (1); and/or
- the device (12) comprises one or more thermohygrometers.

8. The device (12) according to any of previous claims 6-7, which further comprises:
- a backlight source of illumination (14) suitable for partially or totally illuminating the chambers (7, 9) through the base (22); and
- image acquisition means (15) which are adapted to acquire one or more images and/or videos of the analytical chamber (9) and to send said images and/or videos to computer means (16); and
- the computer means (16) configured to provide data acquisition and processing, said computer means (16) controlling both the backlight illumination source (14) and the image acquisition means (15).

9. The device (12) according to any of previous claims 6-8, wherein:
- the size of the disturbance pins (10) is 2 to 8 mm in diameter in case said disturbance pins (10) exhibit a circular geometry and/or;
- the size of the disturbance pins (10) is 2 to 8 mm width in case said disturbance pins (10) have a non-circular geometry; and/or
- at least one of the disturbance pins (10) is located at a distance between 2 and 12 mm from the outlet (5) of the insertion (1).

10. The device (12) according to any of previous claims 6-9, which further comprises a motion platform (17) connected to the measuring cuvette (13), said motion platform (17) being adapted to apply a moving force to the measuring cuvette (13).

11. The device (12) according to any of previous claims 6-10, which further comprises a feeding accessory (18) made partially or totally of an antistatic material, the feeding accessory (18) comprising:
- a lower portion (18.1) geometrically adapted to fit the loading chamber (7) and/or the insertion (1);
- a locking mechanism (18.5);
- a central portion (18.2) comprising an aperture (18.2.1) which can be blocked by the locking mechanism (18.5) and suitable for systematically releasing the powder sample to the lower portion (18.1); and
- an upper portion (18.3) suitable for collecting the powder sample; the upper portion (18.3) being adapted to be screwed into central portion (18.2) and suitable for systematically releasing the powder sample to the central portion (18.2);
- a cap (18.4) adapted to be screwed into the upper portion (18.3), said cap (18.4) being adapted to hold an interchangeable sieve such that said sieve is placed between the cap (18.4) and the upper portion (18.3);
and wherein the locking mechanism (18.5) is adapted to be allocated in the central portion (18.2).

12. The device (12) according to any of the previous claims 6-11 further comprising:
- at least a darkfield source of illumination (19), which in turn comprises tilt means controlled by the computer means (16) and/or manually for adjusting the angle of the darkfield illumination source (19) with regard to the base (6, 11, 22);
- a programmable device (20) configured to switch between the backlight source of illumination (14) and the darkfield source of illumination (19).

13. The device (12) according to any of previous claims 6-12 which further comprises vibration-generating means (21) adapted to be applied to the measuring cuvette (13).

14. A method (100) for characterizing the particle surface of a powder sample; and the method (100) comprising performing the following steps using the device (12) according to any of the previous claims 6-13:
- selecting (101) a preferred insertion (1) geometry and introducing it into the device (12);
- depositing (103) the powder sample into the loading chamber (7);
- performing at least a consolidation step (104) which comprises the compaction of the powder sample;
- applying (105) a moving force to the measuring cuvette (13) to generate a flow of the powder sample that migrates from the loading chamber (7) to the analytical chamber (9); the moving force displacing the measuring cuvette (13) from a home position to an end position;
- when the measuring cuvette (13) reaches the end position, illuminating (106) the measuring cuvette (13);
- acquiring (107) one or more images of the flow pattern of the powder sample at the end position;
- processing (108) the one or more images to obtain one or more parameters characterizing the surface properties of the powder sample; and
- outputting (109) the one or more parameters.

15. The method (100) according to the previous claim, which further comprises:
- operating (110) vibration-generating means (21) to induce a vibration in the measuring cuvette (13) during the migration of the sample of lubricated powder from the loading chamber (7) to the analytical chamber (9); and/or
- performing at least a standardization step by operating the vibration-generating means (21) when the measuring cuvette (13) is placed at the home and/or end positions.

## Patentansprüche

1. Abnehmbarer Einsatz (1) zum Zusammenwirken mit einer Messküvette (13), wobei die Messküvette (13) geeignet ist, eine Pulverprobe aufzunehmen, und der Einsatz (1) Folgendes umfasst:
- einen kuppelförmigen Abschnitt (2), der dazu konfiguriert ist, die Pulverprobe durch einen Einlass (2.1) aufzunehmen;
- einen Trichterabschnitt (4), der mit dem kuppelförmigen Abschnitt (2) verbunden ist und in Strömungsverbindung mit dem kuppelförmigen Abschnitt (2) steht, wobei der Trichterabschnitt (4) einen Auslass (5) umfasst, der an einem engsten Teil des Trichterabschnitts (4) platziert ist, wobei der Auslass (5) dazu konfiguriert ist, eine Analysekammer (9) der Küvette mit der Pulverprobe zu versorgen; und
- einen Rahmen (3), der den kuppelförmigen Abschnitt (2) und den Trichterabschnitt (4) umgibt und integriert; wobei der Einsatz (1) ein inertes Material umfasst.

2. Einsatz (1) nach dem vorhergehenden Anspruch, wobei:
- der Trichterabschnitt (4) einen Akzeptanzwinkel zwischen 15-50° definiert; und/oder
- die Form des kuppelförmigen Abschnitts (2) im Wesentlichen einen Halbkreis aufzeigt oder mindestens ein gekrümmtes Segment umfasst; und/oder
- der Durchmesser des kuppelförmigen Abschnitts (2) im Bereich von 8-18 mm liegt oder, falls er eine Kurve oder ein gekrümmtes Segment umfasst, die Sehne der Kurve oder des Segments im Bereich von 8-18 mm liegt; und/oder
- die Länge des Trichterabschnitts (4) im Bereich von 10-35 mm liegt; und/oder
- der Auslass (5) im Wesentlichen eine viereckige Form, eine kreisförmige Form oder eine Kombination davon aufweist.

3. Messküvette (13) zum Zusammenwirken mit dem abnehmbaren Einsatz (1) der vorhergehenden Ansprüche 1-2, wobei die Messküvette (13) geeignet ist, eine Pulverprobe aufzunehmen, wobei die Messküvette (13) aus inerten Materialien hergestellt ist, wobei die Messküvette (13) eine Basis (22) und einen Satz von zwei Teilen (6, 11) umfasst, die einen oberen Teil (11) und einen unteren Teil (6) umfassen, wobei die Teile (6, 11) miteinander verbunden sind und die Basis (22) enthalten; wobei die Basis (22) aus einem lichtdurchlässigen Material hergestellt ist und dazu angepasst ist, die Pulverprobe unterzubringen; und die Basis (22) dazu konfiguriert ist, Blitze von mindestens einer Beleuchtungsquelle (14, 19) zu empfangen und solche Blitze durch die Basis (22) hindurchzulassen; und wobei der obere Teil (11) eine Ladekammer (7) und eine Analysekammer (9) umfasst; wobei der untere Teil (6) die Basis (22) hält, während er ihre Beleuchtung mittels der mindestens einen Beleuchtungsquelle (14, 19) ermöglicht; und wobei:
- die Ladekammer (7) einen feststehenden Abschnitt (8) umfasst, der angepasst ist, um den Einsatz (1) nach einem der vorhergehenden Ansprüche 1-2 unterzubringen, sodass der Einsatz (1) im Wesentlichen komplementär ist und in den feststehenden Abschnitt (8) passt; und
- die Analysekammer (9) an die Ladekammer (7) angrenzt und mit der Ladekammer (7) über den Auslass (5) des Einsatzes (1) in Strömungsverbindung steht, und wobei die Analysekammer (9) einen oder mehrere Störungsstifte (10) umfasst.

4. Messküvette (13) nach dem vorhergehenden Anspruch 3, wobei:
- die Messküvette (13) und die Basis (22) aus antistatischen Materialien hergestellt sind; und/oder
- mindestens einer der Störungsstifte (10) zylindrisch mit einem Basisdurchmesser zwischen 2 und 8 mm ist, und dieser mindestens eine der Störungsstifte (10) aus rostfreiem Stahl hergestellt ist und dazu angepasst ist, mit dem Auslass (5) des Einsatzes (1) und dem Trichterabschnitt (4) ausgerichtet zu werden.

5. Messküvette (13) nach einem der vorhergehenden Ansprüche 3-4, wobei die Basis (6, 11, 22) eine Platte aus nicht klebendem, lichtdurchlässigen Material, vorzugsweise ein Fluorpolymer wie Polytetrafluorethylen, umfasst.

6. Vorrichtung (12), die zur Analyse einer Pulverprobe geeignet ist, wobei die Vorrichtung (12) den Einsatz (1) nach den vorhergehenden Ansprüchen 1 oder 2 und die Messküvette (13) nach einem der vorhergehenden Ansprüche 3-5 umfasst.

7. Vorrichtung (12) nach dem vorhergehenden Anspruch, wobei:
- die Vorrichtung (12) ferner ein schaltbares Verriegelungssystem (23) umfasst, das dazu angepasst ist, den Auslass (5) des Einsatzes (1) selektiv zu öffnen oder zu blockieren; und/oder
- die Vorrichtung (12) ein oder mehrere Thermohygrometer umfasst.

8. Vorrichtung (12) nach einem der vorhergehenden Ansprüche 6-7, die ferner Folgendes umfasst:
- eine Hintergrundbeleuchtungsquelle (14), die geeignet ist, die Kammern (7, 9) durch die Basis (22) teilweise oder vollständig zu beleuchten; und
- Bilderfassungsmittel (15), die dazu angepasst sind, ein oder mehrere Bilder und/oder Videos der Analysekammer (9) zu erfassen und die Bilder und/oder Videos an Computermittel (16) zu senden; und
- die Computermittel (16), die dazu konfiguriert sind, die Datenerfassung und -verarbeitung bereitzustellen, wobei die Computermittel (16) sowohl die Hintergrundbeleuchtungsquelle (14) als auch die Bilderfassungsmittel (15) steuern.

9. Vorrichtung (12) nach einem der vorhergehenden Ansprüche 6-8, wobei:
- die Größe der Störungsstifte (10) 2 bis 8 mm im Durchmesser für den Fall beträgt, dass die Störungsstifte (10) eine kreisförmige Geometrie aufzeigen, und/oder;
- die Größe der Störungsstifte (10) 2 bis 8 mm Breite für den Fall beträgt, dass die Störungsstifte (10) eine nicht kreisförmige Geometrie aufweisen; und/oder
- mindestens einer der Störungsstifte (10) in einem Abstand zwischen 2 und 12 mm vom Auslass (5) des Einsatzes (1) liegt.

10. Vorrichtung (12) nach einem der vorhergehenden Ansprüche 6-9, die ferner eine Bewegungsplattform (17) umfasst, die mit der Messküvette (13) verbunden ist, wobei die Bewegungsplattform (17) dazu angepasst ist, eine Bewegungskraft auf die Messküvette (13) auszuüben.

11. Vorrichtung (12) nach einem der vorhergehenden Ansprüche 6-10, die ferner ein Zuführungszubehör (18) umfasst, das teilweise oder vollständig aus einem antistatischen Material hergestellt ist, wobei das Zuführungszubehör (18) Folgendes umfasst:
- einen unteren Abschnitt (18.1), der geometrisch dazu angepasst ist, in die Ladekammer (7) und/oder den Einsatz (1) zu passen;
- einen Verriegelungsmechanismus (18.5);
- einen Zentralabschnitt (18.2), umfassend eine Öffnung (18.2.1), die durch den Verriegelungsmechanismus (18.5) blockiert werden kann und geeignet ist, die Pulverprobe systematisch an den unteren Abschnitt (18.1) abzugeben; und
- einen oberen Abschnitt (18.3), der zum Sammeln der Pulverprobe geeignet ist; wobei der obere Abschnitt (18.3) dazu angepasst ist, in den Zentralabschnitt (18.2) geschraubt zu werden und geeignet ist, die Pulverprobe systematisch an den Zentralabschnitt (18.2) abzugeben;
- eine Kappe (18.4), die dazu angepasst ist, in den oberen Abschnitt (18.3) geschraubt zu werden, wobei die Kappe (18.4) dazu angepasst ist, ein austauschbares Sieb zu halten, sodass das Sieb zwischen der Kappe (18.4) und dem oberen Abschnitt (18.3) platziert ist;
und wobei der Verriegelungsmechanismus (18.5) dazu angepasst ist, in dem Zentralabschnitt (18.2) angeordnet zu werden.

12. Vorrichtung (12) nach einem der vorhergehenden Ansprüche 6-11, ferner umfassend:
- mindestens eine Dunkelfeldbeleuchtungsquelle (19), die ihrerseits Neigungsmittel umfasst, die von den Computermitteln (16) und/oder manuell zum Einstellen des Winkels der Dunkelfeldbeleuchtungsquelle (19) in Bezug auf die Basis (6, 11, 22) gesteuert werden;
- eine programmierbare Vorrichtung (20), die dazu konfiguriert ist, zwischen der Hintergrundbeleuchtungsquelle (14) und der Dunkelfeldbeleuchtungsquelle (19) umzuschalten.

13. Vorrichtung (12) nach einem der vorhergehenden Ansprüche 6-12, die ferner Schwingungserzeugungsmittel (21) umfasst, die dazu angepasst sind, an die Messküvette (13) angelegt zu werden.

14. Verfahren (100) zum Charakterisieren der Partikeloberfläche einer Pulverprobe; und wobei das Verfahren (100) das Durchführen der folgenden Schritte unter Verwendung der Vorrichtung (12) nach einem der vorhergehenden Ansprüche 6-13 umfasst:
- Auswählen (101) einer bevorzugten Geometrie des Einsatzes (1) und Einführen desselben in die Vorrichtung (12);
- Einbringen (103) der Pulverprobe in die Ladekammer (7);
- Durchführen mindestens eines Konsolidierungsschritts (104), der die Verdichtung der Pulverprobe umfasst;
- Anlegen (105) einer Bewegungskraft an die Messküvette (13), um einen Fluss der Pulverprobe zu erzeugen, der von der Ladekammer (7) zur Analysekammer (9) wandert; wobei die Bewegungskraft die Messküvette (13) aus einer Ausgangsposition in eine Endposition verschiebt;
- wenn die Messküvette (13) die Endposition erreicht, Beleuchten (106) der Messküvette (13);
- Erfassen (107) eines oder mehrerer Bilder des Fließmusters der Pulverprobe in der Endposition;
- Verarbeiten (108) des einen oder der mehreren Bilder, um einen oder mehrere Parameter zu erlangen, die die Oberflächeneigenschaften der Pulverprobe charakterisieren; und
- Ausgeben (109) des einen oder der mehreren Parameter.

15. Verfahren (100) nach dem vorhergehenden Anspruch, das ferner Folgendes umfasst:
- Betätigen (110) von Schwingungserzeugungsmitteln (21), um eine Schwingung in der Messküvette (13) während der Wanderung der Probe des geschmierten Pulvers von der Ladekammer (7) zur Analysekammer (9) zu induzieren; und/oder
- Durchführen mindestens eines Standardisierungsschritts durch Betätigen der Schwingungserzeugungsmittel (21), wenn die Messküvette (13) in der Ausgangs- und/oder Endposition platziert ist.

## Revendications

1. Insert détachable (1) pour une coopération avec une cuvette de mesure (13), la cuvette de mesure (13) étant appropriée pour la réception d'un échantillon de poudre, et l'insert (1) comprenant :
- une partie en forme de dôme (2), qui est conçue pour recevoir l'échantillon de poudre à travers une entrée (2.1) ;
- une partie entonnoir (4) reliée à la partie en forme de dôme (2) et en communication d'écoulement avec ladite partie en forme de dôme (2), la partie entonnoir (4) comprenant une sortie (5) placée au niveau d'une partie la plus étroite de la partie entonnoir (4), la sortie (5) étant conçue pour alimenter une chambre analytique (9) de ladite cuvette avec l'échantillon de poudre ; et
- un cadre (3) entourant et intégrant la partie en forme de dôme (2) et la partie entonnoir (4) ;
dans lequel l'insert (1) comprend un matériau inerte.

2. Insert (1) selon la revendication précédente, dans lequel :
- la partie entonnoir (4) définit un angle d'acceptation entre 15 et 50 ° ; et/ou
- la forme de la partie en forme de dôme (2) présente sensiblement un demi-cercle ou comprend au moins un segment incurvé ; et/ou
- le diamètre de la partie en forme de dôme (2) est au sein de la plage de 8 à 18 mm ou, dans le cas où elle comprend une courbe ou un segment incurvé, la corde de ladite courbe ou dudit segment est au sein de la plage de 8 à 18 mm ; et/ou
- la longueur de la partie entonnoir (4) est au sein de la plage de 10 à 35 mm ; et/ou
- la sortie (5) présente sensiblement une forme quadrilatérale, une forme circulaire ou une combinaison de celles-ci.

3. Cuvette de mesure (13) pour une coopération avec l'insert détachable (1) selon les revendications précédentes 1 à 2, la cuvette de mesure (13) étant appropriée pour la réception d'un échantillon de poudre, la cuvette de mesure (13) étant faite de matériaux inertes, dans laquelle la cuvette de mesure (13) comprend une base (22) et un ensemble de deux parties (6, 11), comprenant une partie supérieure (11) et une partie inférieure (6), les parties (6, 11) étant jointes ensemble et contenant la base (22) ; dans laquelle la base (22) est faite d'un matériau translucide et adaptée pour accueillir l'échantillon de poudre ; et la base (22) est conçue pour recevoir des éclairs provenant d'au moins une source d'illumination (14, 19) et pour permettre à de tels éclairs de passer à travers la base (22) ; et dans laquelle la partie supérieure (11) comprend une chambre de chargement (7) et une chambre analytique (9) ; et dans laquelle la partie inférieure (6) maintient la base (22) tout en permettant son illumination au moyen de l'au moins une source d'illumination (14, 19) ; et dans laquelle :
- la chambre de chargement (7) comprend une partie fixe (8) adaptée pour accueillir l'insert (1) selon l'une quelconque des revendications précédentes 1 à 2, de telle sorte que l'insert (1) est sensiblement complémentaire de et s'ajuste dans la partie fixe (8) ; et
- la chambre analytique (9) est située adjacente à la chambre de chargement (7) et en communication d'écoulement avec ladite chambre de chargement (7) à travers la sortie (5) de l'insert (1), et dans laquelle la chambre analytique (9) comprend une ou plusieurs broches de perturbation (10).

4. Cuvette de mesure (13) selon la revendication précédente 3, où :
- la cuvette de mesure (13) et la base (22) sont faites de matériaux antistatiques ; et/ou
- au moins une des broches de perturbation (10) est cylindrique avec un diamètre de base entre 2 et 8 mm, et ladite au moins une des broches de perturbation (10) est faite d'acier inoxydable et est adaptée pour être alignée avec la sortie (5) de l'insert (1) et la partie entonnoir (4).

5. Cuvette de mesure (13) selon l'une quelconque des revendications précédentes 3 à 4, dans laquelle la base (6, 11, 22) comprend une plaque de matériau translucide non collant, de préférence un polymère fluoré tel que le polytétrafluoroéthylène.

6. Dispositif (12) approprié pour l'analyse d'un échantillon de poudre, le dispositif (12) comprenant l'insert (1) selon les revendications précédentes 1 ou 2 et la cuvette de mesure (13) selon l'une quelconque des revendications précédentes 3 à 5.

7. Dispositif (12) selon la revendication précédente, dans lequel :
- le dispositif (12) comprend en outre un système de verrouillage commutable (23) qui est adapté pour ouvrir ou bloquer sélectivement la sortie (5) de l'insert (1) ; et/ou
- le dispositif (12) comprend un ou plusieurs thermohygromètres.

8. Dispositif (12) selon l'une quelconque des revendications précédentes 6 à 7, qui comprend en outre :
- une source d'illumination à rétroéclairage (14) appropriée pour l'illumination partielle ou totale des chambres (7, 9) à travers la base (22) ; et
- des moyens d'acquisition d'image (15) qui sont adaptés pour acquérir une ou plusieurs images et/ou vidéos de la chambre analytique (9) et pour envoyer lesdites images et/ou vidéos à des moyens informatiques (16) ; et
- les moyens informatiques (16) conçus pour fournir une acquisition et un traitement de données, lesdits moyens informatiques (16) commandant à la fois la source d'illumination à rétroéclairage (14) et les moyens d'acquisition d'image (15).

9. Dispositif (12) selon l'une quelconque des revendications précédentes 6 à 8, dans lequel :
- la taille des broches de perturbation (10) est de 2 à 8 mm de diamètre dans le cas où lesdites broches de perturbation (10) présentent une géométrie circulaire et/ou ;
- la taille des broches de perturbation (10) est de 2 à 8 mm de largeur dans le cas où lesdites broches de perturbation (10) ont une géométrie non circulaire ; et/ou
- au moins une des broches de perturbation (10) est située à une distance entre 2 et 12 mm de la sortie (5) de l'insert (1).

10. Dispositif (12) selon l'une quelconque des revendications précédentes 6 à 9, qui comprend en outre une plate-forme de mouvement (17) reliée à la cuvette de mesure (13), ladite plate-forme de mouvement (17) étant adaptée pour appliquer une force de mouvement à la cuvette de mesure (13).

11. Dispositif (12) selon l'une quelconque des revendications précédentes 6 à 10, qui comprend en outre un accessoire d'alimentation (18) fait partiellement ou totalement d'un matériau antistatique, l'accessoire d'alimentation (18) comprenant :
- une partie inférieure (18.1) géométriquement adaptée pour s'ajuster à la chambre de chargement (7) et/ou à l'insert (1) ;
- un mécanisme de verrouillage (18.5) ;
- une partie centrale (18.2) comprenant une ouverture (18.2.1) qui peut être bloquée par le mécanisme de verrouillage (18.5) et appropriée pour la libération systématique de l'échantillon de poudre vers la partie inférieure (18.1) ; et
- une partie supérieure (18.3) appropriée pour la collecte de l'échantillon de poudre ; la partie supérieure (18.3) étant adaptée pour être vissée dans la partie centrale (18.2) et appropriée pour la libération systématique de l'échantillon de poudre vers la partie centrale (18.2) ;
- un capuchon (18.4) adapté pour être vissé dans la partie supérieure (18.3), ledit capuchon (18.4) étant adapté pour maintenir un tamis interchangeable de telle sorte que ledit tamis est placé entre le capuchon (18.4) et la partie supérieure (18.3) ;
et dans lequel le mécanisme de verrouillage (18.5) est adapté pour être attribué dans la partie centrale (18.2).

12. Dispositif (12) selon l'une quelconque des revendications précédentes 6 à 11 comprenant en outre :
- au moins une source d'illumination à fond noir (19), qui comprend à son tour des moyens d'inclinaison commandés par les moyens informatiques (16) et/ou manuellement pour l'ajustement de l'angle de la source d'illumination à fond noir (19) par rapport à la base (6, 11, 22) ;
- un dispositif programmable (20) conçu pour commuter entre la source d'illumination à rétroéclairage (14) et la source d'illumination à fond noir (19).

13. Dispositif (12) selon l'une quelconque des revendications précédentes 6 à 12 qui comprend en outre des moyens générateurs de vibration (21) adaptés pour être appliqués à la cuvette de mesure (13).

14. Procédé (100) de caractérisation de la surface de particule d'un échantillon de poudre ; et le procédé (100) comprenant la réalisation des étapes suivantes à l'aide du dispositif (12) selon l'une quelconque des revendications précédentes 6 à 13 :
- la sélection (101) d'une géométrie d'insert (1) préférée et son introduction dans le dispositif (12) ;
- le dépôt (103) de l'échantillon de poudre dans la chambre de chargement (7) ;
- la réalisation d'au moins une étape de consolidation (104) qui comprend le compactage de l'échantillon de poudre ;
- l'application (105) d'une force de mouvement à la cuvette de mesure (13) pour générer un flux de l'échantillon de poudre qui migre de la chambre de chargement (7) à la chambre analytique (9) ; la force de mouvement déplaçant la cuvette de mesure (13) d'une position initiale à une position finale ;
- lorsque la cuvette de mesure (13) atteint la position finale, l'illumination (106) de la cuvette de mesure (13) ;
- l'acquisition (107) d'une ou plusieurs images du schéma d'écoulement de l'échantillon de poudre au niveau de la position finale ;
- le traitement (108) des un ou plusieurs images pour obtenir un ou plusieurs paramètres caractérisant les propriétés de surface de l'échantillon de poudre ; et
- la délivrance (109) des un ou plusieurs paramètres.

15. Procédé (100) selon la revendication précédente, qui comprend en outre :
- le fonctionnement (110) de moyens générateurs de vibration (21) pour induire une vibration dans la cuvette de mesure (13) pendant la migration de l'échantillon de poudre lubrifiée de la chambre de chargement (7) à la chambre analytique (9) ; et/ou
- la réalisation d'au moins une étape de normalisation par le fonctionnement des moyens générateurs de vibration (21) lorsque la cuvette de mesure (13) est placée au niveau des positions initiale et/ou finale.
